(19) Europäisches Patentamt
European Patent Office
Office européen des brevets

(11) EP 4 667 510 A1

(12) EUROPEAN PATENT APPLICATION
published in accordance with Art. 153(4) EPC

(43) Date of publication:
24.12.2025 Bulletin 2025/52

(21) Application number: 24756835.5

(22) Date of filing: 09.02.2024

(51) International Patent Classification (IPC):
C08G 77/38 (2006.01) A61K 8/06 (2006.01)
A61K 8/31 (2006.01) A61K 8/37 (2006.01)
A61K 8/81 (2006.01) A61K 8/89 (2006.01)
A61K 8/92 (2006.01) A61K 8/891 (2006.01)
A61K 8/894 (2006.01) A61Q 1/00 (2006.01)
A61Q 1/02 (2006.01) A61Q 19/00 (2006.01)
C08G 77/46 (2006.01) C08G 77/48 (2006.01)
C08K 5/5415 (2006.01) C08L 83/04 (2006.01)
C08L 83/10 (2006.01) C08L 83/12 (2006.01)
C08L 91/00 (2006.01)

(52) Cooperative Patent Classification (CPC):
A61K 8/06; A61K 8/31; A61K 8/37; A61K 8/81; A61K 8/89; A61K 8/891; A61K 8/894; A61K 8/92; A61Q 1/00; A61Q 1/02; A61Q 19/00; C08G 77/38; C08G 77/46; C08G 77/48; C08K 5/5415; (Cont.)

(86) International application number:
PCT/JP2024/004637

(87) International publication number:
WO 2024/171990 (22.08.2024 Gazette 2024/34)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority: 15.02.2023 JP 2023021356
07.06.2023 JP 2023093829

(71) Applicant: SHIN-ETSU CHEMICAL CO., LTD.
Tokyo 1000005 (JP)

(72) Inventor: IMAI, Taro
Annaka-shi, Gunma 379-0224 (JP)

(74) Representative: Angerhausen, Christoph
Boehmert & Boehmert
Anwaltspartnerschaft mbB
Pettenkoferstrasse 22
80336 München (DE)

# (54) CARBOXY GROUP-MODIFIED ORGANOPOLYSILOXANE HAVING (POLY)GLYCEROL GROUP OR POLYOXYALKYLENE GROUP, AND COSMETIC PREPARATION

(57) Provided are an organopolysiloxane having excellent emulsification stability and a cosmetic preparation including the organopolysiloxane are provided. The organopolysiloxane is a carboxy group-modified organopolysiloxane including a (poly)glycerol group or polyoxyalkylene group represented by the following formula (1):
[Chem. 1]

$$(R_1{}^2SiO_{2/2})_a(R^1R^2SiO_{2/2})_b(R^1R^3SiO_{2/2})_c(R^1R^4SiO_{2/2})_d(R^1SiO_{3/2})_e(SiO_{4/2})_f(R^1{}_3SiO_{1/2})_{2+e+2f} \quad (1)$$

wherein, in the formula, $R^1$ is a monovalent hydrocarbon group, $R^2$ is a (poly)glycerol group or a polyoxyalkylene group, $R^3$ is a carboxy group, and $0 \leq a \leq 100$, $1 \leq b \leq 10$, $1 \leq c \leq 10$, $0 \leq d \leq 10$, $e \geq 0$, and $f \geq 0$.

EP 4 667 510 A1

(52) Cooperative Patent Classification (CPC): (Cont.)
**C08L 83/04; C08L 83/10; C08L 83/12; C08L 91/00**

## Description

Technical Field

The present invention relates to an organopolysiloxane and a cosmetic preparation including the same.

Background Art

Silicone-based surfactants are refreshing and non-sticky and provide excellent sense of use and, therefore, are generally widely used. For example, a large number of emulsified compositions containing polyether-modified silicone or polyglycerol-modified silicone have been proposed (Patent documents 1 and 2). However, when a polar oil such as ester oil is used as the oil agent, the emulsification stability is insufficient, and phase separation occurs over time in some cases.

Patent document 3 proposes a use of carboxy group-modified silicone in an oil-in-water type emulsified composition.

PRIOR ART DOCUMENTS

Patent documents

Patent document 1: JP-A-2021-075488
Patent document 2: WO 2018/117172
Patent document 3: JP-A-2020-172481

Summary of Invention

Technical Problem

However, for example, the emulsification stability may be deteriorated in combined use with zinc oxide microparticles, and there is a room for further improving the emulsification stabilizing ability of silicone-based surfactants.

Accordingly, an increase in the amount of an emulsifier or a thickener for enhancing the emulsification stability rather enhances stickiness, which causes a problem of deteriorating the texture of use that is an advantage of silicone-based surfactants.

The present invention has been made in consideration of the above circumstances, and it is an object to provide an organopolysiloxane having excellent emulsification stability and a cosmetic preparation including the organopolysiloxane.

Solution to Problem

The present inventor has diligently studied in order to achieve the above object, as a result, has found that an emulsified composition obtained by using an organopolysiloxane including a (poly)glycerol group or a polyoxyalkylene group and a carboxy group as an emulsifier has significantly excellent storage stability, and has arrived at the present invention.

That is, the present invention provides carboxy group-modified organopolysiloxanes including a (poly)glycerol group or polyoxyalkylene group shown below and cosmetic preparations including the organopolysiloxanes.

<1> A carboxy group-modified organopolysiloxane represented by following formula (1):
[Chem. 1]

$$(R^1{}_2SiO_{2/2})_a(R^1R^2SiO_{2/2})_b(R^1R^3SiO_{2/2})_c(R^1R^4SiO_{2/2})_d(R^1SiO_{3/2})_e(SiO_{4/2})_f(R^1{}_3SiO_{1/2})_{2+e+2f} \quad (1)$$

wherein, in the formula, $R^1$s are independently a monovalent hydrocarbon group selected from an alkyl group having 1 to 20 carbon atoms, an aryl group having 6 to 15 carbon atoms, and an aralkyl group having 7 to 15 carbon atoms;

$R^2$s are independently a group represented by following formula (2) or (3):

[Chem. 2]

$$-C_gH_{2g}-O-R^5 \qquad (2)$$

wherein, in the formula, $R^5$ is a group in which a hydrogen atom or a monovalent hydrocarbon group having 1 to 30 carbon atoms independently binds to a terminal oxygen atom of the group represented by following formula (2-1):

[Chem. 3]

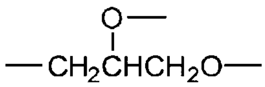 (2-1)

or to a terminal oxygen atom of a plurality of linked groups represented by the formula (2-1), g in the formula (2) is an integer satisfying $0 \leq g \leq 20$, and the number h of the groups represented by the formula (2-1) in $R^5$ is an integer satisfying $1 \leq h \leq 10$;
[Chem. 4]

$$C_iH_{2i}-O\{C_2H_4O\}_{j1}\{C_3H_6O\}_{j2}R6 \qquad (3)$$

wherein, in the formula, $R^6$ is a hydrogen atom or a monovalent hydrocarbon group having 1 to 30 carbon atoms, i is an integer satisfying $0 \leq i \leq 20$, j1 and j2 are integers satisfying $2 \leq j\ 1 \leq 100$ and $0 \leq j2 \leq 100$, respectively, and the order of units grouped by j 1 and units grouped by j2 is arbitrary;

$R^3$s are independently a group represented by following formula (4):
[Chem. 5]

$$-L^1-((OR^7)_k-L^2)_z-COOH \qquad (4)$$

wherein, in the formula, $L^1$ and $L^2$ are each a divalent linking group having 2 to 20 carbon atoms, $R^7$ is a divalent alkylene group having 2 to 4 carbon atoms, k is an integer satisfying $0 \leq k \leq 100$, and z is 0 or 1;
$R^4$s are independently a group represented by following formula (5), (6), (7), or (8):
[Chem. 6]

$$-(CH_2)_2-C_nH_{2n}-(SiR^1{}_2O)_m-SiR^1{}_3 \qquad (5)$$

$$-(CH_2)_2-C_nH_{2n}-SiR^1{}_{m1}-(OSiR^1{}_3)_{3-m1} \qquad (6)$$

$$-(CH_2)_2-C_nH_{2n}-SiR^1{}_{m1}-(OSiR^1{}_{m2}(OSiR^1{}_3)_{3-m2})_{3-m1} \qquad (7)$$

$$-(CH_2)_2-C_nH_{2n}-SiR^1{}_{m1}-(OSiR^1{}_{m2}(OSiR1_{m3}(OSiR^1{}_3)_{3-m3})_{3-m2})_{3-m1} \qquad (8)$$

wherein, in the formulae, $R^1$ is as defined above, n and m are integers satisfying $0 \leq n \leq 5$ and $0 \leq m \leq 100$, respectively, and m1, m2, and m3 are integers satisfying $0 \leq ml \leq 2$, $0 \leq m2 \leq 2$, and $0 \leq m3 \leq 2$; and
a, b, c, d, e, and f are integers satisfying the followings:

$$0 \leq a \leq 100;$$

$$1 \leq b \leq 10;$$

$$1 \leq c \leq 10;$$

$$0 \leq d \leq 10;$$

$$e \geq 0; \text{ and } f \geq 0,$$

and
the order of constituting units grouped by a to f, respectively, is arbitrary.

<2> The carboxy group-modified organopolysiloxane according to <1>, wherein the organopolysiloxane is represented by following formula (1-1):

[Chem. 7]

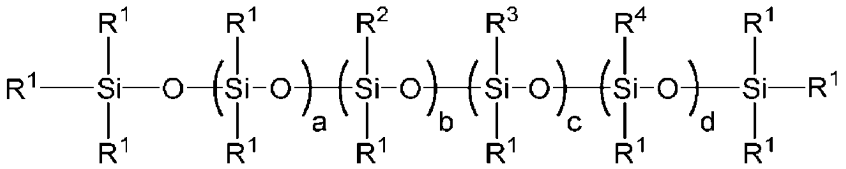

(1-1)

wherein, in the formula, $R^1$, $R^2$, $R^3$, $R^4$, a, b, c, and d are as defined above.

<3> A cosmetic preparation comprising:

(A) a carboxy group-modified organopolysiloxane represented by the formula (1) according to <1> or <2>;
(B) an oil agent; and
(C) water.

<4> The cosmetic preparation according to <3>, wherein the oil agent (B) comprises one or more selected from a silicone oil, a hydrocarbon oil, and a fatty acid ester.

<5> The cosmetic preparation according to <3> or <4>, wherein the cosmetic preparation is an oil-in-water type emulsified cosmetic preparation.

## Advantageous Effects of Invention

An emulsified composition containing the carboxy group-modified organopolysiloxane including a (poly)glycerol group or polyoxyalkylene group of the present invention has little changes in viscosity or particle size over time, and a cosmetic preparation obtained using the emulsified composition has excellent stability.

## Description of Embodiments

The present invention will now be described in detail.

[Carboxy group-modified organopolysiloxane]

The organopolysiloxane of the present invention is a carboxy group-modified organopolysiloxane including a (poly)glycerol group or polyoxyalkylene group represented by the following formula (1): [Chem. 8]

$$(R^1{}_2SiO_{2/2})_a(R^1R^2SiO_{2/2})_b(R^1R^3SiO_{2/2})_c(R^1R^4SiO_{2/2})_d(R^1SiO_{3/2})_e(SiO_{4/2})_f(R^1{}_3SiO_{1/2})_{2+e+2f} \quad (1)$$

wherein, in the formula, $R^1$s are independently a monovalent hydrocarbon group selected from an alkyl group having 1 to 20 carbon atoms, an aryl group having 6 to 15 carbon atoms, and an aralkyl group having 7 to 15 carbon atoms; $R^2$s are independently a group represented by the following formula (2) or (3):
[Chem. 9]

$$-C_gH_{2g}-O-R^5 \quad (2)$$

wherein, in the formula, $R^5$ is a group in which a hydrogen atom or a monovalent hydrocarbon group having 1 to 30 carbon atoms independently binds to a terminal oxygen atom of the group represented by the following formula (2-1):

[Chem. 10]

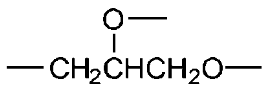
(2-1)

or to a terminal oxygen atom of a plurality of linked groups represented by the formula (2-1), g in the formula (2) is an integer satisfying $0 \leq g \leq 20$, and the number h of the groups represented by the formula (2-1) in $R^5$ is an integer satisfying $1 \leq h \leq 10$;

[Chem. 11]

$$-C_iH_{2i}-O(C_2H_4O)_{j1}(C_3H_6O)_{j2}R^6 \qquad (3)$$

wherein, in the formula, $R^6$ is a hydrogen atom or a monovalent hydrocarbon group having 1 to 30 carbon atoms, i is an integer satisfying $0 \leq i \leq 20$, j1 and j2 are integers satisfying $2 \leq j1 \leq 100$ and $0 \leq j2 \leq 100$, respectively, and the order of units grouped by j1 and units grouped by j2 is arbitrary;

$R^3$s are independently a group represented by the following formula (4):

[Chem. 12]

$$-L^1- ((OR^7)_k-L^2)_z-COOH \qquad (4)$$

wherein, in the formula, $L^1$ and $L^2$ are each a divalent linking group having 2 to 20 carbon atoms, $R^7$ is a divalent alkylene group having 2 to 4 carbon atoms, k is an integer satisfying $0 \leq k \leq 100$, and z is 0 or 1;

$R^4$s are independently a group represented by the following formula (5), (6), (7), or (8):

[Chem. 13]

$$-(CH_2)_2-C_nH_{2n}-(SiR^1{}_2O)_m-SiR^1{}_3 \qquad (5)$$

$$-(CH_2)_2-C_nH_{2n}-SiR^1{}_{m1}-(OSiR^1{}_3)_{3-m1} \qquad (6)$$

$$-(CH_2)_2-C_nH_{2n}-SiR^1{}_{m1}-(OSiR^1{}_{m2}(OSiR^1{}_3)_{3-m2})_{3-m1} \qquad (7)$$

$$-(CH_2)_2-C_nH_{2n-}SiR1_{m1}-(OSiR^1{}_{m2}(OSiR^1{}_{m3}(OSiR^1{}_3)_{3-m3})_{3-m2})_{3-m1} \qquad (8)$$

wherein, in the formulae, $R^1$ is as defined above, n and m are integers satisfying $0 \leq n \leq 5$ and $0 \leq m \leq 100$, respectively, and m1, m2, and m3 are integers satisfying $0 \leq m1 \leq 2$, $0 \leq m2 \leq 2$, and $0 \leq m3 \leq 2$; and

a, b, c, d, e, and f are integers satisfying the followings:

$$0 \leq a \leq 100;$$

$$1 \leq b \leq 10;$$

$$1 \leq c \leq 10;$$

$$0 \leq d \leq 10;$$

$$e \geq 0; \text{ and } f \geq 0,$$

and

the order of constituting units grouped by a to f, respectively, is arbitrary.

In the formula (1), the order of constituting units grouped by a to f, respectively, is arbitrary. The constituting unit group grouped by a, the constituting unit group grouped by b, the constituting unit group grouped by c, the constituting unit group grouped by d, the constituting unit group grouped by e, and the constituting unit group grouped by f may be arranged in any order of the constituting unit groups; and each constituting unit grouped by a, each constituting unit grouped by b, each constituting unit grouped by c, each constituting unit grouped by d, each constituting unit grouped by e, and each

constituting unit grouped by f may be arranged in any order of all of the constituting units.

In the formula (1), $R^1$s are independently a monovalent hydrocarbon group selected from an alkyl group having 1 to 20 carbon atoms, an aryl group having 6 to 15 carbon atoms, and an aralkyl group having 7 to 15 carbon atoms. The alkyl group may be linear or branched or may be cyclic. Specifically, examples of $R^1$ include alkyl groups, such as a methyl group, an ethyl group, a propyl group, a butyl group, a pentyl group, a hexyl group, a heptyl group, an octyl group, a nonyl group, a decyl group, a dodecyl group, a tetradecyl group, and a hexadecyl group; cycloalkyl groups, such as a cyclopentyl group; aryl groups, such as a phenyl group and a tolyl group; and aralkyl groups, such as a benzyl group and a phenethyl group. Each molecule may include different $R^1$s. $R^1$ is preferably a methyl group. When the organopolysiloxane of the formula (1) includes an alkyl group having 6 or more carbon atoms as $R^1$, the amount of the alkyl group having 6 or more carbon atoms is preferably from 5 to 40% by mass, more preferably from 10 to 30% by mass, in the total mass of the organopolysiloxane of the formula (1).

In the formula (1), $R^2$s are independently a group represented by the following formula (2) or (3), and each molecule may include different $R^2$s.

[Chem. 14]

$$-C_gH_{2g}-O-R^5 \qquad (2)$$

[Chem. 15]

$$-C_iH_{2i}-O(C_2H_4O)_{j1}(C_3H_6O)_{j2}R^6 \qquad (3)$$

In the group represented by the formula (2), $R^5$ is a group in which a hydrogen atom or a monovalent hydrocarbon group having 1 to 30 carbon atoms independently binds to a terminal oxygen atom of the group represented by the following formula (2-1):

[Chem. 16]

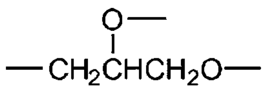

(2-1)

or to a terminal oxygen atom of a plurality of linked groups represented by the formula (2-1).

When a plurality of groups represented by the formula (2-1) is linked, examples of the linking structure include a straight chain structure in which positions 1 or positions 3 of glycerol are linked together and a branched structure in which position 2 of glycerol and position 1 or position 3 of glycerol are linked together. In the branched structure, the number of branching points is not particularly limited, bur is preferably 2 or less or 1 or less.

In the hydrogen atom or the monovalent hydrocarbon group having 1 to 30 carbon atoms that binds to a terminal oxygen atom, examples of the hydrocarbon group include alkyl groups having 1 to 30, preferably 1 to 16, carbon atoms, aryl groups having 6 to 30, preferably 6 to 15, carbon atoms, and aralkyl groups having 7 to 30, preferably 7 to 15, carbon atoms. Specifically, the examples include alkyl groups such as a methyl group, an ethyl group, a propyl group, a butyl group, a pentyl group, a cyclopentyl group, a hexyl group, a cyclohexyl group, a heptyl group, an octyl group, a neopentyl group, a decyl group, a lauryl group, a stearyl group, and an oleyl group; aryl groups such as a phenyl group and a naphthyl group; and aralkyl groups such as a benzyl group and a 2-phenylethyl group.

In the formula (2), g is an integer satisfying $0 \le g \le 20$, and g may be 2 or more or 3 or more. Specifically, $-C_gH_{2g}-$ can be, for example, $-(CH_2)_2-$, $-(CH_2)_3-$, $-CH_2CH(CH_3)CH_2-$, $-(CH_2)_4-$, $-(CH_2)_5-$, $-(CH_2)_6-$, $-(CH_2)_7-$, $-(CH_2)_8-$, $-(CH_2)_2-CH(CH_2CH_2CH_3)-$, or $-CH_2-CH(CH_2CH_3)-$.

The number h of the groups represented by the formula (2-1) in $R^5$ is an integer satisfying $1 \le h \le 10$, preferably $2 \le h \le 5$, and more preferably $2 \le h \le 3$. When h is greater than 10, the organopolysiloxane itself has a high viscosity, and the sense of use and usability are deteriorated.

In the (poly)glycerol group, a monoglycerol group and a polyglycerol group are included, and structural isomers are also included. For example, $R^2$ is a group derived from a monoglycerol of the following general formula (9), a diglycerol of the general formula (10) or (11), or a triglycerol of any of the general formulae (12) to (15), in which g = 3 and h = 1 to 3 in the formula (2).

[Chem. 17]

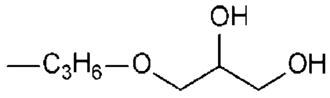

(9)

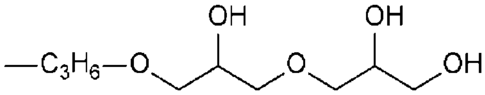

(10)

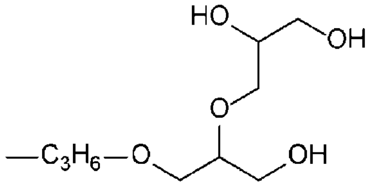

(11)

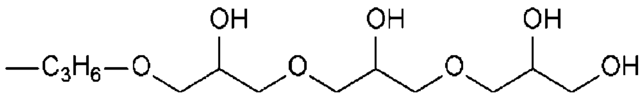

(12)

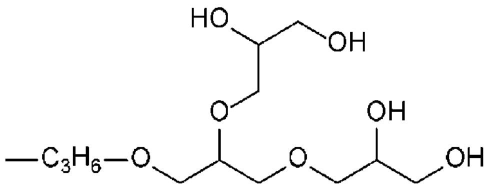

(13)

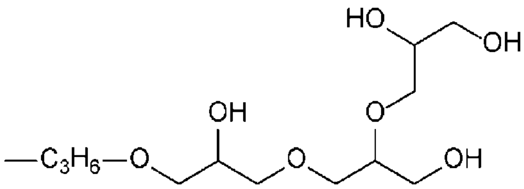

(14)

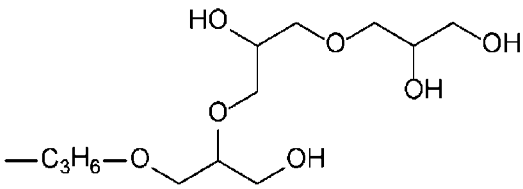

(15)

In the group represented by the formula (3), $R^6$ is a hydrogen atom or a monovalent hydrocarbon group having 1 to 30 carbon atoms. Examples of the hydrocarbon group include alkyl groups having 1 to 30, preferably 1 to 16, carbon atoms, aryl groups having 6 to 30, preferably 6 to 15, carbon atoms, and aralkyl groups having 7 to 30, preferably 7 to 15, carbon atoms. Specifically, the examples include alkyl groups such as a methyl group, an ethyl group, a propyl group, a butyl group, a pentyl group, a cyclopentyl group, a hexyl group, a cyclohexyl group, a heptyl group, an octyl group, a neopentyl group, a decyl group, a lauryl group, a stearyl group, and an oleyl group; aryl groups such as a phenyl group and a naphthyl group; and aralkyl groups such as a benzyl group and a 2-phenylethyl group.

In the formula (3), i is an integer satisfying $0 \le i \le 20$ and may be 2 or more or 3 or more. Specifically, $-C_iH_{2i}-$ can be, for example, $-(CH_2)_2-$, $-(CH_2)_3-$, $-CH_2CH(CH_3)CH_2-$, - $(CH_2)_4-$, $-(CH_2)_5-$, $-(CH_2)_6-$, $-(CH_2)_7-$, $-(CH_2)_8-$, $-(CH_2)_2-CH(CH_2CH_2CH_3)-$, or $-CH_2-CH(CH_2CH_3)-$.

In the formula (3), j1 is an integer satisfying $2 \le j1 \le 100$, preferably $2 \le j1 \le 50$, and more preferably $2 \le j1 \le 15$. and j2 is an integer satisfying $0 \le j2 \le 100$, preferably $0 \le j2 \le 50$, and more preferably $0 \le j2 \le 5$.

The binding order of the oxyalkylene units grouped by j1 and j2 shown in the formula (3) is arbitrary and may be block or random.

When the organopolysiloxane of the present invention is used as an emulsifier, the amount of $R^2$ is preferably from 5 to 70% by mass, more preferably from 10 to 60% by mass, in the total mass of the organopolysiloxane.

In the formula (1), $R^3$s are independently a group represented by following formula (4), and each molecule may include different $R^3$s.
[Chem. 18]

$$-L^1-((OR^7)_k-L^2)_z-COOH \qquad (4)$$

wherein, in the formula, $L^1$ and $L^2$ are each a divalent linking group having 2 to 20 carbon atoms, $R^7$ is a divalent alkylene group having 2 to 4 carbon atoms, k is an integer satisfying $0 \leq k \leq 100$, and z is 0 or 1.
Here, $L^1$ and $L^2$ are each a divalent linking group having 2 to 20 carbon atoms. Examples of the divalent linking group include alkylene groups having 2 to 20, preferably 2 to 10, carbon atoms. Specifically, the examples include a methylene group, an ethylene group, a trimethylene group, a tetramethylene group, a pentamethylene group, a hexamethylene group, a heptamethylene group, an octamethylene group, a decamethylene group, a dodecamethylene group, and an octadecamethylene group.
$R^7$ is a divalent alkylene group having 2 to 4 carbon atoms. Examples of the divalent alkylene group include an ethylene group, a trimethylene group, and a tetramethylene group.
k is an integer satisfying $0 \leq k \leq 100$, preferably $0 \leq k \leq 50$, and more preferably $0 \leq k \leq 5$.
When the organopolysiloxane of the present invention is used as an emulsifier, the amount of $R^3$ is preferably from 1 to 50% by mass, more preferably from 1 to 25% by mass, in the total mass of the organopolysiloxane. Examples of $R^3$ include groups derived from the carboxylic acids of the following general formulae (16) to (18):

[Chem. 19]

$$-C_{10}H_{20}-COOH \qquad (16)$$

$$-C_3H_6-COOH \qquad (17)$$

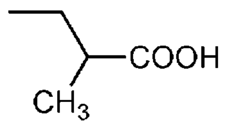

(18)

In the formula (1), $R^4$s are independently a group represented by following general formula (5), (6), (7), or (8), and each molecule may include different $R^4$s.
[Chem. 20]

$$-(CH_2)_2-C_nH_{2n}-(SiR^1{}_2O)_m-SiR^1{}_3 \qquad (5)$$

$$-(CH_2)_2-C_nH_{2n}-SiR^1{}_{m1}-(OSiR^1{}_3)_{3-m1} \qquad (6)$$

$$-(CH_2)_2-C_nH_{2n}-SiR^1{}_{m1}-(OSiR^1{}_{m2}(OSiR^1{}_3)_{3-m2})_{3-m1} \qquad (7)$$

$$-(CH_2)_2-C_nH_{2n}-SiR^1{}_{m1}-(OSiR^1{}_{m2}(OSiR^1{}_{m3}(OSiR^1{}_3)_{3-m3})_{3-m2})_{3-m1} \qquad (8)$$

wherein, in the formulae, $R^1$ is as defined above, n and m are integers satisfying $0 \leq n \leq 5$ and $0 \leq m \leq 100$, respectively, and m1, m2, and m3 are integers satisfying $0 \leq m1 \leq 2$, $0 \leq m2 \leq 2$, and $0 \leq m3 \leq 2$.
n is an integer satisfying $0 \leq n \leq 5$. In particular, in synthesis by a reaction between a vinylsiloxy group and a hydrosilyl group, n is 0. In addition, m is an integer satisfying $0 \leq m \leq 100$, preferably $1 \leq m \leq 30$, and m1, m2, and m3 are integers satisfying $0 \leq m1 \leq 2$, $0 \leq m2 \leq 2$, and $0 \leq m3 \leq 2$.

In the formula (1), a, b, c, d, e, and f are integers satisfying $0 \leq a \leq 100$, $1 \leq b \leq 10$, $1 \leq c \leq 10$, $0 \leq d \leq 10$, $e \geq 0$, and $f \geq 0$, respectively.

a is an integer satisfying $0 \leq a \leq 100$, preferably $1 \leq a \leq 80$, and more preferably $5 \leq a \leq 60$.
b is an integer satisfying $1 \leq b \leq 10$, preferably $1 \leq b \leq 8$, and more preferably $1 \leq b \leq 5$.
c is an integer satisfying $1 \leq c \leq 10$, preferably $1 \leq c \leq 8$, and more preferably $1 \leq c \leq 5$.
d is an integer satisfying $1 \leq d \leq 10$, preferably $1 \leq d \leq 8$, and more preferably $1 \leq d \leq 3$.
e is an integer satisfying $e \geq 0$, preferably $0 \leq e \leq 10$, and more preferably $0 \leq e \leq 5$.

f is an integer satisfying $f \geq 0$, preferably $0 \leq f \leq 10$, and more preferably $0 \leq f \leq 5$.

The organopolysiloxane of the present invention is preferably represented by following formula (1-1):

[Chem. 21]

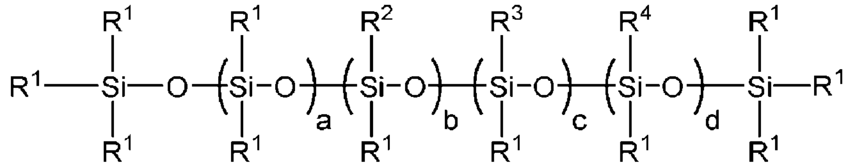

(1-1)

wherein, in the formula, $R^1$, $R^2$, $R^3$, $R^4$, a, b, c, and d are as defined above.

The organopolysiloxane of the present invention represented by the formula (1) can be synthesized by hydrosilylation reaction of an organohydrogen polysiloxane (H) including a hydrosilyl group, which is a reaction active point; a compound including an alkenyl group (carbon-carbon unsaturated bond), which becomes a raw material of $R^2$ and $R^3$ in the formula (1), at a terminal; and optionally a compound including an alkenyl group, which becomes a raw material of $R^1$ and $R^4$, at a terminal.

Examples of the organohydrogen polysiloxane (H) including a hydrosilyl group as a reaction active point include compounds in which $R^2$, $R^3$, and $R^4$ in the above formula (1-1) are substituted with hydrogen atoms, and a part of $R^1$s is optionally substituted with a hydrogen atom or atoms.

The carboxyl group represented by the formula (4), which is a characteristic of the present invention, can be introduced by hydrosilylation reaction of the organohydrogen polysiloxane (H) with a compound represented by the following formula (ii) having an alkenyl group, which becomes a raw material of $R^3$, at the terminal.

[Chem. 22]

(ii) $CH_2=CH-R^{3'}$

$R^{3'}$ is a group that becomes $R^3$ from $-CH_2CH_2R^{3'}$. That is, $R^{3'}$ includes a carboxy group, and the carboxy group in $R^{3'}$ is preferably substituted with a protection group such as a trimethylsilyl group, a t-butyl group, and a benzyl group. The substitution of the carboxy group in $R^{3'}$ with a protecting group is preferable because the side reaction or the catalytic inhibition of the carboxyl group can be prevented in the hydrosilylation reaction with the organohydrogen polysiloxane (H).

The (poly)glycerol group or polyoxyalkylene group represented by the formula (2) or (3) can be introduced by hydrosilylation reaction of the organohydrogen polysiloxane (H) with a compound represented by the following formula (iii) having an alkenyl group, which becomes a raw material of $R^2$, at the terminal.

[Chem. 23]

(i i i) $CH_2=CH-R^{2'}$

$R^{2'}$ is a group that becomes $R^2$ from $-CH_2CH_2R^{2'}$.

A part of $R^1$s in the formula (1) can be introduced by hydrosilylation reaction of the organohydrogen polysiloxane (H) with a compound represented by the following formula (iv) having an alkenyl group, which becomes a raw material of $R^1$, at the terminal.

[Chem. 24]

(i v) $CH_2=CH-R^{1'}$

$R^{1'}$ is a group that becomes $R^1$ from $-CH_2CH_2R^{1'}$.

The group represented by the formula (5), (6), (7), or (8) can be introduced by hydrosilylation reaction of the organohydrogen polysiloxane (H) with any of the compounds represented by the following formulae (v) having an alkenyl group, which becomes a raw material of $R^4$, at the terminal.

[Chem. 25]

(v) $CH_2=CH-C_nH_{2n}-(SiR^1{}_2O)_m-SiR^1{}_3$ $CH_2=CH-C_nH_{2n}-SiR^1{}_{m1}-(OSiR^1{}_3)_{3-m1}$ $CH_2=CH-C_nH_{2n}-SiR^1{}_{m1}-(OSiR^1{}_{m2}(OSiR^1{}_3)_{3-m2})_{3-m1}$ $CH_2=CH-C_nH_{2n}-SiR^1{}_{m1}-(OSiR^1{}_{m2}(OSiR^1{}_{m3}(OSiR^1{}_{m3})_{3-m3})_{3-m2})_{3-m1}$

As the hydrosilylation reaction, a known method can be adopted. The organopolysiloxane of the present

invention can be manufactured by adjusting the configuration in the formula (1) by the raw materials to be used and their amounts. For example, the organohydrogen polysiloxane (H), compounds represented by the formulae (ii) and (iii), and optionally compounds represented by the formulae (iv) and (v) are added to a reaction vessel at a target molar ratio, and a platinum catalyst is further added thereto for reaction.

The addition order of the compounds represented by the formulae (ii), (iii), (iv), and (v) is not particularly limited, but, for example, the compounds of the formulae (iv), (ii), (v), and (iii) can be added in this order.

The platinum catalyst is not particularly limited, and examples thereof include $PtCl_4$, $H_2PtCl_6 \cdot 6H_2O$, a Pt-ether complex, a Pt-olefin complex, $PdCl_2(PPh_3)_2$, $PdCl_2(PhCN)_2$, $RhCl_2(PPh_3)_3$ (in these formulae, Ph is a phenyl group), and complexes of platinum chloride, chloroplatinic acid, or chloroplatinate with a vinyl group-containing siloxane.

These catalysts may be used alone or as a mixture of two or more.

Among them, particularly preferred is Karstedt catalyst (a complex of 1,1,3,3-tetramethyl-1,3-divinyldisiloxane and chloroplatinic acid neutralized with sodium bicarbonate.

The amount of the platinum catalyst is not particularly limited and may be a catalytic amount. The catalytic amount is an amount that is sufficient for proceeding the hydrosilylation reaction and is, for example, preferably 0.02 parts by mass or less, more preferably from 0.00001 to 0.02 parts by mass, further preferably from 0.0001 to 0.01 parts by mass, and particularly preferably from 0.0003 to 0.005 parts by mass as the platinum metal atom equivalent amount of the platinum catalyst with respect to 100 parts by mass of the organohydrogen polysiloxane (H).

The reaction can be performed at, for example, from 50°C to 90°C, but is not particularly limited thereto.

A solvent is preferably used for the reaction. The solvent is not particularly limited, and examples thereof include isopropyl alcohol, tetrahydrofuran, and toluene.

When a product obtained by substituting a carboxy group with a protecting group such as a trimethylsilyl group is used as the compound represented by the formula (ii), deprotection is performed. The deprotection can be performed by, for example, heating in the coexistence of alcohol.

[Cosmetic preparation]

The cosmetic preparation of the present invention contains:

a carboxy group-modified organopolysiloxane (A) represented by the formula (1);
an oil agent (B), and
water (C).

In the present invention, the form of the cosmetic preparation is a composition.

<Component (A)>

The component (A) is a carboxy group-modified organopolysiloxane including a (poly)glycerol group or polyoxyalkylene group represented by the formula (1), and a single type or a combination of two or more types of organopolysiloxanes can be used.

The amount of the component (A) is not particularly limited, but is preferably from 0.1 to 30% by mass, more preferably from 0.1 to 15% by mass, of the total amount of the cosmetic preparation.

<Component (B)>

The component (B) of the present invention is an oil agent. The oil agent (B) of the present invention may be any of a liquid, a solid, and a semi-solid, and examples thereof include a hydrocarbon oil, a higher fatty acid, a higher alcohol, natural animal and vegetable oils and fats and semisynthetic fat and oil, an ester oil, a silicone oil, and a fluorine-based oil agent. These oil agents can be used alone or in combination of two or more.

Examples of the hydrocarbon oil include linear or branched hydrocarbon oils, which may be volatile hydrocarbon oils or involatile hydrocarbon oils. Specifically, the examples include an olefin oligomer, isododecane (nomenclature (INCI: Isododecane)), dodecane (nomenclature (INCI: Dodecane)), isohexadecane (nomenclature (INCI: Isohexadecane)), undecane (nomenclature (INCI: Undecane)), squalane (nomenclature (INCI: Squalane)), squalene (nomenclature (INCI: Squalene)), a mineral oil (nomenclature (INCI: Mineral Oil)), liquid isoparaffin, polyisobutylene (nomenclature), hydrogenated polyisobutene (nomenclature (INCI: Hydrogenated Polyisobutene)), and (C13-15) alkane (nomenclature (INCI: C13-15 Alkane)).

Examples of the higher fatty acid include oleic acid (nomenclature (INCI: Oleic Acid)), linoleic acid (nomenclature (INCI: Linoleic Acid)), linolenic acid (nomenclature (INCI: Linolenic Acid)), arachidonic acid (nomenclature (INCI: Arachidonic Acid)), eicosapentaenoic acid (EPA) (nomenclature (INCI: Eicosapentaenoic Acid)), docosahexaenoic acid

(DHA) (nomenclature (INCI: Docosahexaenoic Acid)), isostearic acid (nomenclature (INCI: Isostearic Acid)), and hydroxystearic acid (nomenclature (INCI: Hydroxystearic Acid)).

Examples of the higher alcohol include linear saturated alcohols having 6 or more carbon atoms, such as lauryl alcohol (INCI), hexyldecanol (INCI), oleyl alcohol (INCI), isostearyl alcohol (INCI), octyldodecanol (INCI), decyltetradecanol (INCI), myristyl alcohol (INCI), cetyl alcohol (INCI), stearyl alcohol (INCI), and behenyl alcohol (INCI); and butyl alcohol (INCI), and also include sterols such as cholesterol (INCI), sitosterol (nomenclature (INCI: Beta-Sitosterol)), phytosterol (INCI), and lanosterol (INCI).

Examples of the natural animal and vegetable oils and fats and semisynthetic fat and oil include avocado oil (nomenclature (INCI: Persea Gratissima (Avocado) Oil)), linseed oil (nomenclature (INCI: Linum Usitatissimum (Linseed) Seed Oil)), almond oil (nomenclature (INCI: Prunus Amygdalus Dulcis (Sweet Almond) Oil)), olive oil (nomenclature (INCI: Olea Europaea (Olive) Fruit Oil)), California nutmeg oil (nomenclature (INCI: Torreya Californica (California Nutmeg) Oil)), citronella oil (nomenclature (INCI: Cymbopogon Nardus (Citronella) Oil)), shark liver oil (nomenclature (INCI: Shark Liver Oil)), cod liver oil (nomenclature (INCI: Cod Liver Oil)), fish liver oil (nomenclature (INCI: Fish Liver Oil)), apricot oil (nomenclature (INCI: Kyounin Yu)), sesame oil (nomenclature (INCI: Sesamum Indicum (Sesame) Seed Oil)), rice germ oil (nomenclature (INCI: Oryza Sativa (Rice) Germ Oil)), rice bran oil (nomenclature (INCI: Oryza Sativa (Rice) Bran Oil)), Camellia Kissi seed oil (nomenclature (INCI: Camellia Kissi Seed Oil)), safflower oil (nomenclature (INCI: Carthamus Tinctorius (Safflower) Seed Oil)), turtle oil (nomenclature (INCI: Turtle Oil)), camellia oil (nomenclature (INCI: Camellia Japonica Seed Oil)), evening primrose oil (nomenclature (INCI: Oenothera Biennis (Evening Primrose) Oil)), corn germ oil (nomenclature (INCI: Zea Mays (Corn) Germ Oil)), rapeseed oil (nomenclature (INCI: RAPE SHUSHI YU)), wheat germ oil (nomenclature (INCI: Triticum Vulgare (Wheat) Germ Oil)), apricot kernel oil (nomenclature (INCI:)), palm oil (nomenclature (INCI: Elaeis Guineensis (Palm) Oil)), palm kernel oil (nomenclature (INCI: Elaeis Guineensis (Palm) Kernel Oil)), castor oil (nomenclature (INCI: Ricinus Communis (Castor) Seed Oil)), hydrogenated castor oil (nomenclature), sunflower oil (nomenclature (INCI: Helianthus Annuus (Sunflower) Seed Oil)), grape seed oil (nomenclature (INCI: Vitis Vinifera (Grape) Seed Oil)), jojoba oil (nomenclature (INCI: Simmondsia Chinensis (Jojoba) Seed Oil)), macadamia nut oil (nomenclature (INCI: Macadamia Ternifolia Seed Oil)), mink oil (nomenclature (INCI: Mink Oil)), meadowfoam oil (nomenclature (INCI: Limnanthes Alba (Meadowfoam) Seed Oil)), cotton seed oil (nomenclature (INCI: Gossypium Herbaceum (Cotton) Seed Oil)), coconut oil (nomenclature (INCI: Cocos Nucifera (Coconut) Oil)), hydrogenated coconut oil (nomenclature (INCI: Hydrogenated Coconut Oil)), and egg yolk oil (nomenclature (INCI:Egg Oil)).

The ester oil is a liquid oil having a form obtained by condensation of a fatty acid having 1 to 20 carbon atoms and an alcohol having 1 to 20 carbon atoms, and examples thereof include monoesters and polyesters such as a diester and a triester. Specifically, the examples include diisobutyl adipate (nomenclature (INCI: Diisobutyl Adipate)), dihexyldecyl adipate (nomenclature), diheptylundecyl adipate (nomenclature (INCI: Diheptylundecyl Adipate)), n-alkylglycol monoisostearate such as isostearyl isostearate (nomenclature (INCI: Isostearyl Isostearate)), isocetyl isostearate (nomenclature (INCI: Isocetyl Isostearate)), trimethylolpropane triisostearate (nomenclature (INCI: Trimethylolpropane Triisostearate)), glycol diethylhexanoate (nomenclature (INCI: Glycol Diethylhexanoate)), cetyl ethylhexanoate (nomenclature (INCI: Cetyl Ethylhexanoate)), triethylhexanoin (nomenclature (INCI: Triethylhexanoin)), trimethylolpropane triethylhexanoate (nomenclature (INCI: Trimethylolpropane Triethylhexanoate)), pentaerythrityl tetraethylhexanoate (nomenclature (INCI: Pentaerythrityl Tetraethylhexanoate)), cetyl octanoate (nomenclature (INCI: Cetyl Ethylhexanoate)), octyldodecyl esters such as octyldodecyl stearoyl stearate (nomenclature (INCI: Octyldodecyl Stearoyl Stearate)), oleyl oleate (nomenclature (INCI: Oleyl Oleate)), octyldodecyl oleate (nomenclature (INCI: Octyldodecyl Oleate)), decyl oleate (nomenclature (INCI: Decyl Oleate)), neopentyl glycol dioctanoate (nomenclature (INCI: Neopentyl Glycol Diethylhexanoate)), neopentyl glycol dicaprate (nomenclature (INCI: Neopentyl Glycol Dicaprate)), triethyl citrate (nomenclature (INCI: Triethyl Citrate)), diethylhexyl succinate (nomenclature (INCI: Diethylhexyl Succinate)), amyl acetate (nomenclature (INCI: Amyl Acetate)), ethyl acetate (nomenclature (INCI: Ethyl Acetate)), butyl acetate (nomenclature (INCI: Butyl Acetate)), isocetyl stearate (nomenclature (INCI: Isocetyl Stearate)), butyl stearate (nomenclature (INCI: Butyl Stearate)), diisopropyl sebacate (nomenclature (INCI: Diisopropyl Sebacate)), diethylhexyl sebacate (nomenclature (INCI: Diethylhexyl Sebacate)), cetyl lactate (nomenclature (INCI: Cetyl Lactate)), myristyl lactate (nomenclature (INCI: Myristyl Lactate)), isononyl isononanoate (nomenclature (INCI: Isononyl Isononanoate)), isotridecyl isononanoate (nomenclature (INCI: Isotridecyl Isononanoate)), palmitate esters such as isopropyl palmitate (nomenclature (INCI: Isopropyl Palmitate)), ethylhexyl palmitate (nomenclature (INCI: Ethylhexyl Isopalmitate)), and hexyldecyl palmitate (nomenclature (INCI: Isocetyl Palmitate, Hexyldecyl Palmitate)), cholesteryl hydroxystearate (nomenclature (INCI: Cholesteryl Hydroxystearate)), myristate esters such as isopropyl myristate (nomenclature (INCI: Isopropyl Myristate)), octyldodecyl myristate (nomenclature (INCI: Octyldodecyl Myristate)), and myristyl myristate (nomenclature (INCI: Myristyl Myristate)), ethylhexyl laurate (nomenclature (INCI: Ethylhexyl Laurate)), hexyl laurate (nomenclature (INCI: Hexyl Laurate)), dioctyldodecyl lauroyl glutamate (nomenclature (INCI: Dioctyldodecyl Lauroyl Glutamate)), lauroyl sarcosine isopropyl ester (nomenclature (INCI: Isopropyl Lauroyl Sarcosinate)), diisostearyl malate (nomenclature (INCI: Diisostearyl Malate)), and glyceride oils such as glyceryl acetate (nomenclature (INCI: Glyceryl Acetate)), glyceryl stearate (nomenclature (INCI: Glyceryl Stearate)), and tri(caprylic/capric) glyceryl (nomenclature (INCI: Caprylic/Capric Triglyceride)).

Examples of the silicone oil include trisiloxane (nomenclature (INCI: Trisiloxane)), volatile dimethicone (nomenclature (INCI: Dimethicone)), low-viscosity dimethicone (nomenclature (INCI: Dimethicone)), cyclotetrasiloxane (nomenclature (INCI: Cyclotetrasiloxane)), cyclopentasiloxane (nomenclature (INCI: Cyclopentasiloxane)), cyclohexasiloxane (nomenclature (INCI: Cyclohexasiloxane)), methyl trimethicone (nomenclature (INCI: Methyl Trimethicone)), caprylyl methicone (nomenclature (INCI: Caprylyl Methicone)), phenyl trimethicone (nomenclature (INCI: Phenyl Trimethicone)), methylphenyl polysiloxane (nomenclature (INCI: Diphenyl Dimethicone)), diphenylsiloxy phenyl trimethicone (nomenclature (INCI: Diphenylsiloxy Phenyl Trimethicone)), ethyl methicone (nomenclature (INCI: Ethyl Methicone)), ethyl trisiloxane (nomenclature (INCI: Ethyl Trisiloxane)), and low to high viscosity linear and branched dimethicone such as hydrogen dimethicone (nomenclature (INCI: Hydrogen Dimethicone)), amodimethicone (nomenclature (INCI: Amodimethicone)), aminopropyl dimethicone (nomenclature (INCI: Aminopropyl Dimethicone)), highly polymerized gummy dimethicone (nomenclature (INCI: Dimethicone)), gummy amodimethicone (nomenclature (INCI: Amodimethicone)), silicone rubber such as a gummy dimethylsiloxane-methylphenyl siloxane copolymer, and cyclic organopolysiloxane solutions of silicone gum and rubber, amino acid-modified silicone, fluorine-modified silicone, and dissolved products of a silicone resin and a silicone resin.

Examples of the fluorine-based oil agent include perfluoropolyethers such as polyperfluoromethylisopropyl ether (nomenclature (INCI: Polyperfluoromethylisopropyl Ether)); and perfluorocarbons such as perfluorodecalin (nomenclature (INCI: Perfluorodecalin)) and perfluorohexane (nomenclature (INCI: Perfluorohexane)).

The component (B) is preferably one or more selected from a silicone oil, a hydrocarbon oil, and a fatty acid ester.

The amount of the component (B) is not particularly limited, but is preferably from 1 to 50% by mass, more preferably from 1 to 25% by mass, of the total amount of the cosmetic preparation.

<Component (C)>

The component (C) of the present invention is water, and examples thereof include, in addition to purified water and distilled water of fruits and plants which are generally used in cosmetic preparations, sea water (INCI: Sea Water), hot spring water (Onsen-Sui), and peat water (INCI: Peat Water) defined by nomenclatures.

The amount of the water (C) is not particularly limited, but is preferably from 5 to 95% by mass, more preferably from 40 to 80% by mass, of the total amount of the cosmetic preparation.

<Component (D)>

The cosmetic preparation of the present invention can further include one or two or more polyhydric alcohols as needed, in addition to the above components (A) to (C).

Examples of the polyhydric alcohol include ethylene glycol, propylene glycol, dipropylene glycol, 1,3-butylene glycol, pentylene glycol, glycerol, diglycerol, triglycerol, and polyglycerol. In particular, 1,3-butylene glycol, propylene glycol, diglycerol, glycerol, and a combination thereof are preferable.

When the component (D) is included, the amount thereof is not particularly limited, but is preferably from 1 to 50% by mass, more preferably from 1 to 25% by mass, of the total amount of the cosmetic preparation.

The cosmetic preparation of the present invention can include optional components described later within a range that does not impair the effects of the present invention.

The shape of the cosmetic preparation of the present invention is not particularly limited, and examples thereof include liquid, cream, paste, solid, mousse, spray, gel, and emulsion forms. The application is also not particularly limited, and the cosmetic preparation can be used as, for example, a skin care cosmetic preparation, a makeup cosmetic preparation, a hair cosmetic preparation, or a sunscreen cosmetic preparation. Specifically, examples of the cosmetic preparation include makeup cosmetic preparations containing the above-described cosmetic preparation components, such as skin care, hair care, and cleansing products, a makeup base, a liquid foundation, a cream foundation, a solid foundation, a blusher, an eyeshadow, a mascara, an eyeliner, an eyebrow, and a lipstick; and UV protection cosmetic preparations containing the above-described cosmetic preparation components, such as a sunscreen emulsion and a sunscreen cream.

The form of the cosmetic preparation of the present invention is not particularly limited, and the cosmetic preparation can also be applied to, for example, any of an oily cosmetic preparation, an aqueous cosmetic preparation, an oil-in-water type emulsified cosmetic preparation, and a water-in-oil type emulsified cosmetic preparation, and a preferable form is an oil-in-water type emulsified cosmetic preparation. In an oil-in-water type form, the organopolysiloxane of the component (A) stabilizes the emulsification interface and provides an emulsified composition with excellent stability over time. Consequently, the makeup durability and the sense of use are improved.

<Optional component>

The cosmetic preparation of the present invention may further include optional components that are usually used in the cosmetic field. Examples of the optional component include an ultraviolet absorber, a surfactant other than the component (A), a film-forming agent, an antiperspirant, an antibacterial agent, a preservative, a fragrance, a salt, an antioxidant, a moisturizer, a pH adjuster, a chelating agent, a refreshing agent, an anti-inflammatory agent, a skin-beautifying agent (such as a whitening agent, a cell activator, a skin roughness-improving agent, a blood circulation promoter, a skin astringent, and an anti-seborrheic agent), vitamins, amino acids, a nucleic acid, hormones, a clathrate compound, a powder, an organic resin, and a thickener. These components may be used alone or in combination of two or more at a suitable amount.

The ultraviolet absorber is not particularly limited as long as it is a raw material that can be usually blended with cosmetic preparations, and a single type or a combination of two or more types of ultraviolet absorbers can be used. Specifically, examples of oil-soluble ultraviolet absorbers include homosalate (nomenclature (INCI: Homosalate)), octocrylene (nomenclature (INCI: Octocrylene)), t-butyl methoxydibenzoylmethane (nomenclature (INCI: Butyl Methoxydibenzoylmethane)), ethylhexyl salicylate (nomenclature (INCI: Ethylhexyl Salicylate)), diethylamino hydroxybenzoyl hexyl benzoate (nomenclature (INCI: Diethylamino Hydroxybenzoyl Hexyl Benzoate)), oxybenzone-6 (nomenclature (INCI: Benzophenone-6)), oxybenzone-9 (nomenclature (INCI: Benzophenone-9)), oxybenzone-1 (nomenclature (INCI: Benzophenone-1)), polysilicone-15 (nomenclature (INCI: Polysilicone-15)), octyl dimethoxybenzylidene dioxoimidazolidine propionate (nomenclature (INCI: Ethylhexyl Dimethoxybenzylidene Dioxoimidazolidine Propionate)), oxybenzone-2 (nomenclature (INCI: Benzophenone-2)), terephthalylidene dicamphor sulfonic acid (nomenclature (INCI: Terephthalylidene Dicamphor Sulfonic Acid)), ethylhexyl triazone (nomenclature (INCI: Ethylhexyl Triazone), methyl bis(trimethylsiloxy)silylisopentyl trimethoxycinnamate (nomenclature (INCI: Isopentyl Trimethoxycinnamate Trisiloxane)), drometrizole trisiloxane (nomenclature (INCI: Drometrizole Trisiloxane)), ethylhexyl dimethyl PABA (nomenclature (INCI: Ethylhexyl Dimethyl PABA)), isopropyl paramethoxycinnamate (nomenclature (INCI: Isopropyl Methoxycinnamate)), ethylhexyl methoxycinnamate (nomenclature (INCI: Ethylhexyl Methoxycinnamate)), bis-ethylhexyloxyphenol methoxyphenyl triazine (nomenclature (INCI: Bis-Ethylhexyloxyphenol Methoxyphenyl Triazine)), oxybenzone-3 (nomenclature (INCI: Benzophenone-3)), oxybenzone-4 (nomenclature (INCI: Benzophenone-4)), oxybenzone-5 (nomenclature (INCI: Benzophenone-5)), phenylbenzimidazole sulfonic acid (nomenclature (INCI: Phenylbenzimidazole Sulfonic Acid)), methylene bis-benzotriazolyl tetramethylbutylphenol (nomenclature (INCI: Methylene Bis-Benzotriazolyl Tetramethylbutylphenol)), glyceryl ethylhexanoate dimethoxycinnamate (nomenclature (INCI: Glyceryl Ethylhexanoate Dimethoxycinnamate)), glyceryl PABA (nomenclature (INCI: Glyceryl PABA)), methyl diisopropylcinnamate (nomenclature (INCI: Diisopropyl Methyl Cinnamate)), cinoxate (nomenclature (INCI: Cinoxate)), and ethylhexyl dimethoxybenzylidene dioxoimidazolidine propionate (nomenclature (INCI: Ethylhexyl Dimethoxybenzylidene Dioxoimidazolidine Propionate)). In addition, an UVA absorber (e.g., hexyl diethylaminohydroxybenzoyl benzoate (nomenclature (INCI: Diethylamino Hydroxybenzoyl Hexyl Benzoate))) and an UVB absorber (e.g., ethylhexyl methoxycinnamate (nomenclature (INCI: Ethylhexyl Methoxycinnamate))) can be used in combination, and the both can be used in arbitrary combination.

In particular, preferred are one or two or more oil-soluble ultraviolet absorbers selected from ethylhexyl methoxycinnamate (nomenclature (INCI: Ethylhexyl Methoxycinnamate)), hexyl diethylaminohydroxybenzoyl benzoate (nomenclature (INCI: Diethylamino Hydroxybenzoyl Hexyl Benzoate)), octyl salicylate (nomenclature (INCI: Ethylhexyl Salicylate)), polysilicone-15 (nomenclature (INCI: Polysilicone-15)), t-butyl methoxydibenzoylmethane (nomenclature (INCI: Butyl Methoxydibenzoylmethane), oxybenzone (nomenclature (INCI: Benzophenone-6)), methylene bis-benzotriazolyl tetramethylbutylphenol (nomenclature (INCI: Methylene Bis-Benzotriazolyl Tetramethylbutylphenol)), bis-ethylhexyloxyphenol methoxyphenyl triazine (nomenclature (INCI: Bis-Ethylhexyloxyphenol Methoxyphenyl Triazine)), homosalate (nomenclature (INCI: Homosalate)), and octocrylene (nomenclature (INCI: Octocrylene)).

The surfactants other than the component (A) are nonionic, anionic, cationic, and amphoteric active agents and are not particularly limited, and any surfactant can be used as long as it is used in general cosmetic preparations. Among these surfactants, preferred are partially cross-linked polyether-modified silicone, partially cross-linked polyglycerol-modified silicone, linear or branched polyoxyethylene-modified organopolysiloxane, linear or branched polyoxyethylene polyoxypropylene-modified organopolysiloxane, linear or branched polyoxyethylene-alkyl co-modified organopolysiloxane, linear or branched polyoxyethylene polyoxypropylene-alkyl co-modified organopolysiloxane, linear or branched polyglycerol-modified organopolysiloxane, linear or branched polyglycerol-alkyl co-modified organopolysiloxane, and pyrrolidone-modified organopolysiloxane. In these surfactants, the amount of the hydrophilic polyoxyethylene group, polyoxyethylene polyoxypropylene group, or polyglycerol residue is preferably from 10 to 70% by mass in the molecule. When partially cross-linked polyether-modified silicone or partially cross-linked polyglycerol-modified silicone is used, in a composition consisting of the cross-linked organopolysiloxane and an oil agent that is liquid at 25°C, the cross-linked organopolysiloxane preferably swells when exposed to a liquid oil, absorbing more than its own weight of the liquid oil agent. As the liquid oil agent, the liquid silicone in oil agents of optional components, a hydrocarbon oil, an ester oil, a natural animal and vegetable oil, a semisynthetic oil, and a fluorine-based oil can be used, and examples thereof include

low-viscosity silicone having a kinematic viscosity of 0.65 to 100 $mm^2/s$ at 25°C, hydrocarbon oils such as liquid paraffin, squalane, isododecane, and isohexadecane, glyceride oils such as trioctanoin, ester oils such as isotridecyl isononanoate, N-acyl glutamic acid ester, and lauroyl sarcosinic acid ester, and natural animal and vegetable oils such as macadamia nut oil. Specific examples include products manufactured by Shin-Etsu Chemical Co., Ltd.: KSG-210, KSG-240, KSG-310, KSG-320, KSG-330, KSG-340, KSG-320Z, KSG-350Z, KSG-710, KSG-810, KSG-820, KSG-830, KSG-840, KSG-820Z, KSG-850Z, and so on. Specific examples of the surfactant other than the cross-linked organopolysiloxane include products manufactured by Shin-Etsu Chemical Co., Ltd.: KF-6011, KF-6013, KF-6043, KF-6017, KF-6028, KF-6038, KF-6048, KF-6100, KF-6104, KF-6105, KF-6106, and so on. In any case, the amount of the surfactant is preferably from 0.1 to 20% by mass of the total amount of the cosmetic preparation. When the amount is 0.1% by mass or more, the dispersing and emulsifying functions can be sufficiently achieved, and an amount of 20% by mass or less is preferable because there is no risk of sticky sense of use of the cosmetic preparation. The HLB of the surfactant is not limited, but is preferably from 2 to 14.5 from the purpose of maintaining the water resistance of the cosmetic preparation.

The film-forming agent is mixed mainly for the purpose of further maintaining the effect sustainability of cosmetic preparations. It is not particularly limited, but a silicone-based composition is preferable from the viewpoint of imparting water repellency. Specifically, trimethylsiloxysilicate (nomenclature (trimethylsiloxysilicate (INCI)), an acryl-silicone coating agent, silicone-modified norbornene, silicone-modified pullulan, and so on can be used. The film-forming agent may be dissolved in an oil agent that is liquid at room temperature in advance and then may be mixed into a cosmetic preparation. As the liquid oil agent, the liquid silicone in oil agents of optional components, a hydrocarbon oil, an ester oil, a natural animal and vegetable oil, a semisynthetic oil, and a fluorine-based oil can be used, and examples thereof include low-viscosity silicone having a kinematic viscosity of 0.65 to 100 $mm^2/s$ at 25°C, hydrocarbon oils such as liquid paraffin, squalane, isododecane, and isohexadecane, glyceride oils such as trioctanoin, ester oils such as isotridecyl isononanoate, N-acyl glutamic acid ester, and lauroyl sarcosinic acid ester, and natural animal and vegetable oils such as macadamia nut oil. Specific examples thereof include products manufactured by Shin-Etsu Chemical Co., Ltd.: KF-7312J, which is a silicone solution of trimethylsiloxysilicate, KP-545 and KP-549, which are silicone solutions of acryl-silicone coating agent, NBN-30-ID, which is an isododecane solution of silicone-modified norbornene, TSPL-30-ID, which is an isododecane solution of silicone-modified pullulan, and TSPL-30-D5, which is a silicone solution.

When the cosmetic preparation according to the present invention is a deodorant agent, an antiperspirant can be optionally blended therein. The antiperspirant is not particularly limited as long as it is a component that reduces perspiration by astringing the skin, and general-purpose components can be widely used, and examples thereof include aluminum chlorohydrate, aluminum chloride, aluminum chlorohydroxy allantoinate, aluminum allantoinate, tannic acid, potassium aluminum sulfate, zinc oxide, zinc paraphenolsulfonate, burnt alum, aluminum zirconium tetrachlorohydrate, and aluminum zirconium trichlorohydrex glycine. In particular, the component of expressing a high effect is preferably an anti-perspiration active component selected from the group consisting of aluminum halides, aluminum hydroxyhalides, and their complexes or mixtures with zirconyl oxyhalides and zirconyl hydroxyhalides. These antiperspirants can be used by being dissolved in water, or the powder thereof can be used by being directly blended with the formulation. The antiperspirant may be a commercially available product. The commercially available product to be used may be in a form of a mixture raw material with another component. The amount of the antiperspirant is not particularly limited and can be appropriately changed depending on the amount of another component. From the purpose of obtaining a deodorant agent having an excellent deodorant effect and the purpose of obtaining a deodorant agent having reduced stimulation on the skin, the amount of the antiperspirant to be blended is preferably from 0.001 to 30% by mass, more preferably from 0.01 to 20% by mass, of the total amount of the cosmetic preparation.

The antibacterial agent is not particularly limited as long as it is a component that provides a deodorizing effect by suppressing the proliferation of skin resident flora producing substances that cause body odor. For example, antibacterial agents, such as triclosan, benzalkonium chloride, benzothonium chloride, chlorhexidine hydrochloride, chlorhexidine gluconate, Halocarban, and isomethylphenol, are generally used. Alternatively, antibacterial essential oil, extract, and so on derived from herbal medicine, such as green tea dry distillation extract, may be blended. Examples of the antibacterial agent having a deodorizing effect, such as essential oil and extract derived from herbal medicine, include Green tea extract, Lavender extract, Scutellaria root extract, Coptis japonica extract, Plantain extract, Artemisia capillaris extract, Aloe arborescens extract, Sophora arborescens root extract, Sasa veitchii leaf extract, Garlic extract, Hamamelis virginiana extract, Black tea extract, Sage leaf extract, Zanthoxylum extract, Ginger root extract, Calamus root extract, Hedera helix extract, Houttuynia extract, Peach fruit extract, Peach leaf extract, Peppermint leaf extract, Cnidium extract, Eucalyptus leaf extract, Peanut seed coat extract, Lychee extract, and Sanguinea oleracea extract.

Examples of the preservative include paraoxybenzoic acid alkyl ester, benzoic acid, sodium benzoate, sorbic acid, potassium sorbate, and phenoxyethanol, and examples of the antibacterial agent include benzoic acid, salicylic acid, carbolic acid, sorbic acid, paraoxybenzoic acid alkyl ester, parachlorometacresol, hexachlorophene, trichlorocarbanilide, and a photosensitizer.

In the fragrance, natural fragrances and synthetic fragrances are included. As the natural fragrances, there are botanical fragrances separated from flowers, leaves, woods, peels, and so on and animal fragrances such as musk and

civet. Examples of the synthetic fragrance include hydrocarbons such as monoterpene; alcohols such as aliphatic alcohols and aromatic alcohols; aldehydes such as terpene aldehydes and aromatic aldehydes; ketones such as alicyclic ketones; esters such as terpenic esters; lactones; phenols; oxides; nitrogen-containing compounds; and acetals.

Examples of the salt include an inorganic salt, an organic acid salt, an amine salt, and an amino acid salt. Examples of the inorganic salt include sodium salts, potassium salts, magnesium salts, calcium salts, aluminum salts, zirconium salts, and zinc salts of inorganic acids such as hydrochloric acid, sulfuric acid, carbonic acid, and nitric acid. Examples of the organic acid salt include salts of organic acids such as acetic acid, dehydroacetic acid, citric acid, malic acid, succinic acid, ascorbic acid, and stearic acid. Examples of the amine salt and amino acid salt include salts of amines such as triethanolamine and salts of amino acids such as glutamic acid. In addition, it is also possible to use salts of hyaluronic acid, chondroitin sulfate, and so on, complexes including an aluminum zirconium glycine complex, and further acid-alkali neutral salts that are used in cosmetic preparation prescriptions.

Examples of the antioxidant include carotenoid, ascorbic acid and its salt, ascorbyl stearate, tocopherol acetate, tocopherol, p-t-butylphenol, butylhydroxyanisole, dibutylhydroxytoluene, phytic acid, ferulic acid, thiotaurine, hypotaurine, sulfite, erythorbic acid and its salt, chlorogenic acid, epicatechin, epigallocatechin, epigallocatechin gallate, apigenin, campherol, myricetin, and quercetin.

Examples of the moisturizer include glucose, xylitol, maltitol, polyethylene glycol, hyaluronic acid, chondroitin sulfate, pyrrolidone carboxylate, polyoxyethylene methyl glucoside, polyoxypropylene methyl glucoside, egg yolk lecithin, soy lecithin, phosphatidylcholine, phosphatidylethanolamine, phosphatidylserine, phosphatidylglycerol, phosphatidylinositol, and a sphingophospholipid.

Examples of the pH adjuster include lactic acid, citric acid, glycolic acid, succinic acid, tartaric acid, dl-malic acid, potassium carbonate, sodium hydrogen carbonate, and ammonium hydrogen carbonate.

Examples of the chelating agent include alanine, sodium edetate, sodium polyphosphate, sodium metaphosphate, phosphoric acid, aspartic acid diacetate, and ethylenediamine disuccinic acid.

Examples of the refreshing agent include L-menthol and camphor.

Examples of the anti-inflammatory agent include allantoin, glycyrrhizic acid and its salt, glycyrrhetinic acid and stearyl glycyrrhetinate, tranexamic acid, and azulene.

Examples of the skin-beautifying agent include whitening agents such as a vitamin C derivative, hydroquinone, tranexamic acid, arbutin, phenylethyl resorcinol, kojic acid, and a plant extract; cell activators such as royal jelly, a photosensitizer, a cholesterol derivative, and a young calf blood extract; skin roughness-improving agents; blood circulation promoters such as nonylic acid vanillylamide, nicotinic acid benzyl ester, nicotinic acid β-butoxyethyl ester, capsaicin, zingerone, cantharis tincture, ichthammol, caffeine, tannic acid, α-borneol, tocopherol nicotinate, inositol hexanicotinate, cyclandelate, cinnarizine, tolazoline, acetylcholine, verapamil, cepharanthine, and γ-oryzanol; skin astringents such as zinc oxide and tannic acid; and anti-seborrheic agents such as sulfur and thianthrol.

Examples of the vitamin include vitamin A group including vitamin A oil, retinol, retinol acetate, and retinol palmitate; vitamin $B_2$ group including riboflavin, riboflavin butyrate, and a flavin adenine nucleotide; vitamin $B_6$ group including pyridoxine hydrochloride, pyridoxine dioctanoate, and pyridoxine tripalmitate; vitamin B group including vitamin $B_{12}$ and its derivative and vitamin $B_{15}$ and its derivative; vitamin C group including L-ascorbic acid, L-ascorbic acid dipalmitic acid ester, L-ascorbic acid-2-sodium sulfate, and dipotassium L-ascorbic acid phosphoric acid diester; vitamin D group including ergocalciferol and cholecalciferol; vitamin E group including α-tocopherol, β-tocopherol, γ-tocopherol, dl-α-tocopherol acetate, dl-α-tocopherol nicotinate, and dl-α-tocopherol succinate; nicotinic acids such as nicotinic acid, benzyl nicotinate, and nicotinamide; pantothenic acids such as vitamin H, vitamin P, calcium pantothenate, D-pantothenyl alcohol, pantothenyl ethyl ether, and acetyl pantothenyl ethyl ether.

Examples of the amino acid include glycine, valine, leucine, isoleucine, serine, threonine, phenylalanine, arginine, lysine, aspartic acid, glutamic acid, cystine, cysteine, methionine, and tryptophane.

Examples of the nucleic acid include a deoxyribonucleic acid.

Examples of the hormone include estradiol and ethinylestradiol.

Examples of the clathrate compound include cyclodextrin.

Examples of the powder include a metal oxide microparticle powder, a color pigment, an inorganic powder, a metal powder, an organic powder, and an inorganic-organic complex powder.

The metal oxide microparticle powder is one or two or more selected from titanium oxide microparticle (nomenclature (INCI: Titanium Dioxide)), iron-containing titanium oxide, zinc oxide (nomenclature (INCI: Zinc Oxide)), cerium oxide (nomenclature (INCI: Cerium Oxide)), and complexes thereof. These metal oxides may be a complex powder with another powder.

The color pigment is not particularly limited as long as it is a pigment that is usually used for the purpose of coloring cosmetic preparations, and it is possible to use any of red iron oxide (nomenclature (INCI: Iron Oxides)), yellow iron oxide (nomenclature (INCI: Iron Oxides)), white titanium oxide (nomenclature (INCI: Titanium Dioxide)), black iron oxide (nomenclature (INCI: Iron Oxides)), ultramarine (nomenclature (INCI: Ultramarines)), Prussian blue (nomenclature (INCI: Ferric Ferrocyanide, Ferric Ammonium Ferrocyanide)), manganese violet (nomenclature (INCI: Manganese Violet)),

cobalt titanate (nomenclature (INCI: Cobalt Titanium Oxide)), chromium hydroxide (nomenclature (INCI: Chromium Hydroxide Green)), chromium oxide (nomenclature (INCI: Chromium Oxide Greens)), aluminum cobalt oxide (nomenclature (INCI: Cobalt Aluminum Oxide)), cobalt titanate (nomenclature (INCI: Cobalt Titanium Oxide)), (titanium/titanium oxide) burned product (nomenclature (INCI: Titanium/Titanium Dioxide)), lithium cobalt titanate (nomenclature (INCI: Lithium Cobalt Titanate)), cobalt titanate (nomenclature (INCI: Cobalt Titanium Oxide)), (iron oxide/titanium oxide) sintered product (nomenclature (IRON OXIDE/TITANIUM DIOXIDO SINTER)), a composite doped with a different metal, such as iron oxide-doped titanium oxide (nomenclature (INCI: Iron Oxides, Titanium Dioxide)), titanium nitride (nomenclature (INCI: Titanium Nitride)), ferrous hydroxide (nomenclature (INCI: Iron Hydroxide)), an inorganic brown pigment such as γ-iron oxide, an inorganic yellow pigment such as ocher, and color pigments such as a laked tar dye and a laked natural dye.

The color pigment is preferably a pigment having a particle diameter, e.g., a volume-average particle diameter, within a range of 150 to 600 nm from the point of view of concealing power, The volume-average particle diameter can be measured with a TEM or the like. When the diameter is less than 150 nm, the concealing power is low, which may decrease the coloring efficiency of cosmetic preparations. When the diameter is greater than 600 nm, the sense of use may be deteriorated. Furthermore, the pigment according to the present invention may be partially or entirely surface-treated with an inorganic compound such as alumina (nomenclature (INCI: Alumina)), aluminum hydroxide (nomenclature (INCI: Aluminum Hydroxide)), silica (nomenclature (INCI: Silica)), and hydrous silica (nomenclature (INCI: Hydrated Silica)).

Examples of the inorganic powder include microparticles made of zirconium oxide (nomenclature (INCI: Zirconium Dioxide)), zinc oxide (nomenclature (INCI: Zinc Oxide)), cerium oxide (nomenclature (INCI: Cerium Oxide)), magnesium oxide (nomenclature (INCI: Magnesium Oxide)), barium sulfate (nomenclature (INCI: Barium Sulfate)), calcium sulfate (nomenclature (INCI: Calcium Carbonate)), magnesium sulfate (nomenclature (INCI: Magnesium Sulfate)), calcium carbonate (nomenclature (INCI: Calcium Carbonate)), magnesium carbonate (nomenclature (INCI: Magnesium Carbonate)), talc (nomenclature (INCI: Talc)), cleaved talc (nomenclature (INCI: Talc)), mica (nomenclature (INCI: Mica)), kaolin (nomenclature (INCI: Kaolin)), sericite (nomenclature (INCI: Mica)), synthetic fluorphlogopite (nomenclature (INCI: Synthetic Fluorphlogopite), biotite (nomenclature (INCI: Biotite), potassium silicate (nomenclature (INCI: Potassium Silicate)), silica (nomenclature (INCI: Silica), fumed silica, aluminum silicate (nomenclature (INCI: Aluminum Silicate)), magnesium silicate (nomenclature (INCI: Magnesium Silicate)), aluminum magnesium silicate (nomenclature (INCI: Magnesium Aluminum Silicate)), calcium silicate (nomenclature (INCI: Calcium Silicate)), aluminum calcium sodium silicate (nomenclature (INCI: Aluminum Calcium Sodium Silicate)), lithium magnesium sodium silicate (nomenclature (INCI: Lithium Magnesium Sodium Silicate)), sodium magnesium silicate (nomenclature (INCI: Sodium Magnesium Silicate)), calcium aluminum borosilicate (nomenclature (INCI: Calcium Aluminum Borosilicate)), calcium sodium borosilicate (nomenclature (INCI: Calcium Sodium Borosilicate)), hydroxyapatite (nomenclature (INCI: Hydroxyapatite)), bentonite (nomenclature (INCI: Bentonite)), montmorillonite (nomenclature (INCI: Montmorillonite)), hectorite (nomenclature (INCI: Hectorite)), zeolite (nomenclature (INCI: Zeolite)), alumina (nomenclature (INCI: Alumina)), aluminum hydroxide (nomenclature (INCI: Aluminum Hydroxide)), boron nitride (nomenclature (INCI: Boron Nitride)), or glass (nomenclature (INCI: Glass)). Examples of inorganic color pearl pigments include pearl agents such as titanium oxide-coated mica; and pearl pigments such as bismuth oxychloride (nomenclature (INCI: Bismuth Oxychloride)), bismuth oxychloride (nomenclature (INCI: Bismuth Oxychloride)) coated with titanium oxide (nomenclature (INCI: Titanium Dioxide)), talc (nomenclature (INCI: Talc)) coated with titanium oxide (nomenclature (INCI: Titanium Dioxide)), argentine, and color mica coated with titanium oxide (nomenclature (INCI: Titanium Dioxide)).

Examples of the metal powder include metal microparticles made of aluminum (nomenclature (INCI: Aluminum Powder)), copper (nomenclature (INCI: Copper Powder)), and silver (nomenclature (INCI: Silver Powder)).

Examples of the organic powder include powders made of silicone, polyamide, polyacrylic acid-acrylic acid ester, polyester, polyethylene, polypropylene, polystyrene, a styrene-acrylic acid copolymer, a divinylbenzene-styrene copolymer, polyurethane, a vinyl resin, a urea resin, a melanin resin, benzoguanamine, polymethylbenzoguanamine, tetrafluoroethylene, polymethyl methacrylate (e.g., methyl polymethacrylate), cellulose, silk, nylon, a phenol resin, an epoxy resin, carbonate, and so on. In particular, examples of the silicone include silicone resin particles (specifically, polymethylsilsesquioxane (nomenclature (INCI: Polymethylsilsesquioxane)) and so on) and silicone resin-coated silicone rubber powders (specifically, (vinyl dimethicone/methicone silsesquioxane) crosspolymer (nomenclature (INCI: Vinyl Dimethicone/Methicone Silsesquioxane Crosspolymer)), (diphenyl dimethicone/vinyl diphenyl dimethicone/silsesquioxane) crosspolymer (nomenclature (INCI: Diphenyl Dimethicone/Vinyl Diphenyl Dimethicone/Silsesquioxane Crosspolymer)), polysilicone-1 crosspolymer (nomenclature (INCI: Polysilicone-1 Crosspolymer)), and polysilicone-22 (nomenclature (INCI: Polysilicone-22)). In addition, metal soap is also included, and examples thereof include powders made of zinc stearate (nomenclature (INCI: Zinc Stearate)), aluminum stearate (nomenclature (INCI: Aluminum Stearate)), calcium stearate (nomenclature (INCI: Calcium Stearate)), magnesium stearate (nomenclature (INCI: Magnesium Stearate)), zinc myristate (nomenclature (INCI: Zinc Myristate)), magnesium myristate (nomenclature (INCI: Magnesium Myristate)), sodium zinc cetyl phosphate (nomenclature (INCI: Sodium Zinc Cetyl Phosphate)), potassium cetyl phosphate (nomenclature (INCI: Potassium Cetyl Phosphate)), and so on. Furthermore, organic dyes are also included, and

examples thereof include tar dyes such as Red No. 3, Red No. 104(1) (nomenclature (INCI: Red 28, Red 28 Lake)), Red No. 106, Red No. 201 (nomenclature (INCI: Red 6)), Red No. 202 (nomenclature (INCI: Red 7)), Red No. 204, Red No. 205, Red No. 220 (nomenclature (INCI: Red 34)), Red No. 226 (nomenclature (INCI: Red 30)), Red No. 227 (nomenclature (INCI: Red 33, RED 33 Lake )), Red No. 228 (nomenclature (INCI: Red 36)), Red No. 230(1) (nomenclature (INCI: Red 22, Red 22 Lake)), Red No. 230(2), Red No. 401, Red No. 505, Yellow No. 4 (nomenclature (INCI: Yellow 5)), Yellow No. 5 (nomenclature (INCI: Yellow 6, Yellow 6 Lake)), Yellow No. 202(1) (nomenclature (INCI: Yellow 8)), Yellow No. 203 (nomenclature (INCI: Yellow 10, Yellow 10 Lake)), Yellow No. 204 (nomenclature (INCI: Yellow 11)), Yellow No. 401 (nomenclature (INCI:)), Blue No. 1 (nomenclature (INCI: Blue 1, Blue 1 Lake)), Blue No. 2, Blue No. 201, Blue No. 205 (nomenclature (INCI: Blue 4)), Blue No. 404, Green No. 3 (nomenclature (INCI: Green 3, Green 3 Lake)), Green No. 201 (nomenclature (INCI: Green 5)), Green No. 202 (nomenclature (INCI: Green 6)), Green No. 204 (nomenclature (INCI: Green 8)), Green No. 205, Orange No. 201 (nomenclature (INCI: Orange 5)), Orange No. 203 (nomenclature (INCI: Pigment Orange 5)), Orange No. 204, Orange No. 205 (nomenclature (INCI: Orange 4, Orange 4 Lake)), Orange No. 206 (nomenclature (INCI: Orange 10)), and Orange No. 207 (nomenclature (INCI: Orange 11)); and natural dyes such as cochineal (nomenclature (INCI: Cochineal)), laccaic acid (nomenclature (INCI: Laccaic Acid)), safflower red (nomenclature (INCI: Carthamus Tinctorius (Safflower) Flower Extract)), Lithospermum Erythrorhizon Root extract (nomenclature (INCI: Lithospermum Officinale Root Extract)), Gardenia yellow, and Gardenia blue (nomenclature (INCI: Hydrolyzed Gardenia Florida Extract)).

Examples of the inorganic-organic complex powder include a complex powder in which the surface of an inorganic powder is coated with an organic powder by a known method.

The powder may be hydrophobized. The hydrophobization agent for surface treatment of the raw material powder is not particularly limited as long as it can impart hydrophobicity, and examples thereof include a silicone treatment agent, waxes, paraffines, an organic fluorine compound of a perfluoroalkyl and a phosphate or the like, a surfactant, an amino acid such as N-acyl glutamic acid, and metal soap such as aluminum stearate (nomenclature (INCI: Aluminum Stearate)) and magnesium myristate (nomenclature (INCI: Magnesium Myristate)).

In particular, a silicone treatment agent is preferably used, and examples thereof include silanes or silylating agents such as triethoxycaprylylsilane (nomenclature (INCI: Triethoxycaprylylsilane)) and trimethoxysilyl dimethicone (nomenclature (INCI: Trimethoxysilyl Dimethicone)); silicone oils such as dimethicone (nomenclature (INCI: Dimethicone)), hydrogen dimethicone (nomenclature (INCI: Hydrogen Dimethicone)), and triethoxysilylethyl polydimethylsiloxyethyl hexyl dimethicone (nomenclature (INCI: Triethoxysilylethyl Polydimethylsiloxyethyl Hexyl Dimethicone)); and silicone compounds such as an (acrylates/tridecyl acrylate/triethoxysilylpropyl methacrylate/dimethicone methacrylate) copolymer (nomenclature (INCI: Acrylates/Tridecyl Acrylate/Triethoxysilylpropyl Methacrylate/Dimethicone Methacrylate Copolymer)) and an (acrylates/dimethicone) copolymer (nomenclature (INCI: Acrylates/Dimethicone Copolymer)). The above-mentioned surface hydrophobization agents can be used alone or in combination of two or more.

Examples of the hydrophobic powder are mentioned below, but the present invention is not limited thereto.

The hydrophobized metal oxide microparticle powder may be a commercially available product. For example, the titanium oxide microparticle is commercially available under the trade names such as STR-100C-LP, STR-100A-LP, STR-100W, STR-100W-LP, STR-100W-OTS, STR-100C-LF, and STR-40-LP (manufactured by Sakai Chemical Industry Co., Ltd.), MT-01, MT-05, MT-100Z, MT-100TV, MT-100AQ, MT-150EX, MT-500B, MT-505SAS, MT-700B, MT-014Z, and SMT-500SAS (manufactured by TAYCA Corporation), and ST-455, ST-455WS, ST-457ECS, and ST-495M (manufactured by Titan Kogyo, Ltd.). Zinc oxide microparticle is commercially available under the trade names such as FINEX-50S-LP2, FINEX-30S-LP2, FINEX-50W, FINEX-30W, FINEX-50W-LP2, FINEX-52W-LP2, FINEX-30W-LP2, FINEX-33W-LP2, FINEX-50-LPT, FINEX-25-LPT, FINEX-50S-LPT, FINEX-30S-LPT, and FINEX-30-OTS (manufactured by Sakai Chemical Industry Co., Ltd.) and MZ-150, MZ-200, MZ-300, MZ-306X, MZ-500HP, MZ-505T, MZ-506X, MZY-203S, MZY-210M3S, TMZ-HA1, and MZX-5080TS (manufactured by TAYCA Corporation), commercially available under the trade names such as (manufactured by TAYCA Corporation).

Examples of the hydrophobized color pigment include KTP-09 series, in particular, KTP-09W, KTP-09R, KTP-09Y, KTP-09B, and so on (manufactured by Shin-Etsu Chemical Co., Ltd.).

## EXAMPLES

The present invention will now be specifically described by showing Examples and Comparative Examples, but the present invention is not limited to the following Examples.

### Synthesis Example

Organopolysiloxanes were obtained by mixing the components shown in Table 1 below at molar ratios shown in Table 1 by the method shown below.

[Table 1]

| | | Synthesis Example 1 | Synthesis Example 2 | Synthesis Example 3 | Synthesis Example 4 | Synthesis Example 5 |
|---|---|---|---|---|---|---|
| (A) Organopolysiloxane | | A-1 | A-2 | A-3 | A-4 | A-5 |
| (H) | H-1 | 1 | - | - | - | - |
| | H-2 | - | 1 | - | - | - |
| | H-3 | - | - | 1 | - | - |
| | H-4 | - | - | - | 1 | - |
| | H-5 | - | - | - | - | 1 |
| (ii) | ii-1 | 1 | 2 | 2 | 1 | 1 |
| (iii) | iii-1 | 4 | - | 2 | - | 3 |
| | iii-2 | - | 1 | - | - | - |
| | iii-3 | - | - | - | 1 | - |
| (iv) | iv-1 | 4 | - | - | - | - |
| | iv-2 | - | - | 6 | - | - |
| (v) | v-1 | - | - | 2 | - | - |
| Numerical values shown in the table show molar ratios. | | | | | | |

Components shown in Table 1 above are as follows:

[Chem. 26]

（H－１）

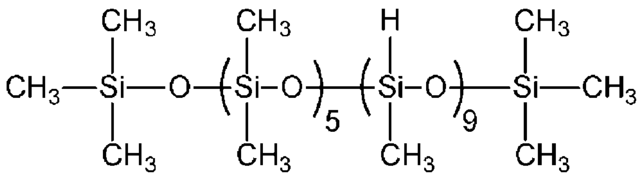

（H－２）

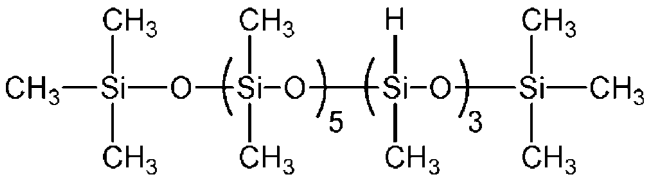

（H－３）

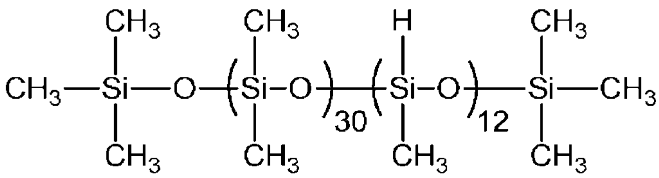

（H－４）

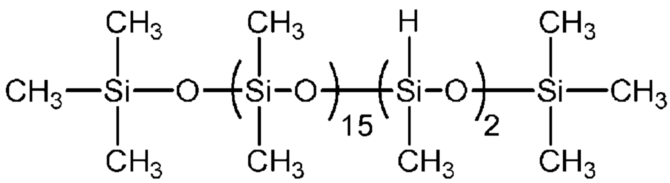

（H－５）

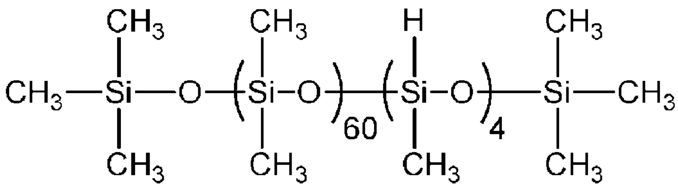

[Chem. 27]

（ｉｉ－１）

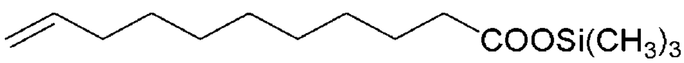

[Chem. 28]

(iii-1)

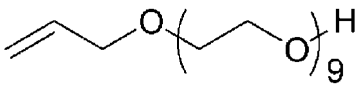

(iii-2)

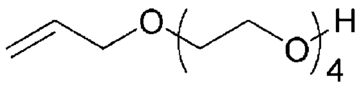

(iii-3)

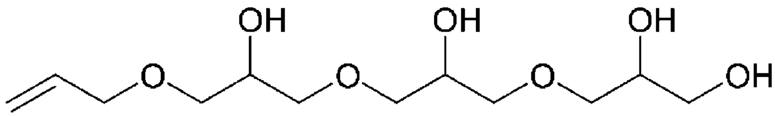

[Chem. 29]

(iv-1) $CH_2=CH-C_{10}H_{21}$

(iv-2) $CH_2=CH-C_{14}H_{21}$

[Chem. 30]

(v-1)

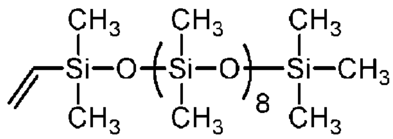

In Synthesis Examples 1 to 5 below, components organohydrogen polysiloxane (H), trimethylsilyl undecylenate (ii), alkene (iv), and organopolysiloxane (v) shown in Table 1 were fed in a reaction vessel, and a platinum catalyst was added thereto for reaction. Subsequently, (poly)glycerol monoallyl ether/polyoxyalkylene monoallyl ether (iii) was added to the reaction vessel, a platinum catalyst was further added for reaction, and then deprotection of undecylenic acid was performed.

[Synthesis Example 1]

Methyl hydrogen polysiloxane (H), trimethylsilyl undecylenate (ii), and alkene (iv) were fed in a reaction vessel, and a platinum catalyst (a silicone oil solution of Karstedt catalyst [3% by mass as the platinum metal equivalent]) was further added thereto in an amount of 3 ppm based on the methyl hydrogen polysiloxane (H), and reaction was performed at 80°C. Subsequently, (poly)glycerol monoallyl ether/polyoxyalkylene monoallyl ether (iii) was added to the reaction vessel, and isopropyl alcohol in an amount of 40% by mass of the total and a platinum catalyst (a silicone oil solution of Karstedt catalyst [3% by mass as the platinum metal equivalent]) in an amount of 3 ppm based on the methyl hydrogen polysiloxane (H) were further added thereto, and reaction was performed at 80°C. Then, methanol was added thereto in an amount of 30% by mass of the total, followed by heating at 60°C to deprotect undecylenic acid. Furthermore, methanol and isopropyl alcohol were removed by heating under reduced pressure to obtain a carboxy group-modified organopolysiloxane (A-1) including a polyglycerol group or a polyoxyalkylene group of Synthesis Example 1 in which in the formula (1), a = 9, b = 4, c = 1, d = 0, e = 0, and f = 0, in the formula (3), i = 3 and j1 = 9, and in the formula (4), $L^1$ is $-C_{10}H_{20}-$. In (A-1), the content of $R^2$ was 49% by mass, and the content of $R^3$ was 5% by mass.

It was verified by $^1$H-NMR measurement that (A-1) has a structure represented by the following structural formula. The content of $R^1$ having 6 or more carbon atoms in (A-1) was 18% by mass. $^1$H-NMR: 0.00 ppm (75H), 0.49 ppm (18H), 0.88 ppm (12H), 1.22 ppm (96H), 1.63 ppm (2H), 2.34 ppm (2H), 3.85 ppm (152H).

[Chem. 31]

(A − 1)

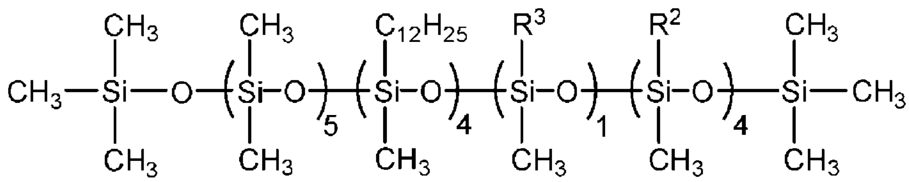

$R^3$: $-C_{10}H_{20}COOH$
$R^2$: $-C_3H_6O(C_2H_4O)_9H$

[Synthesis Example 2]

Organopolysiloxane (A-2) in which in the formula (1), a = 5, b = 1, c = 2, d = 0, e = 0, and f = 0, in the formula (3), i = 3 and j1 = 4, and in the formula (4), $L^1$ is $-C_{10}H_{20}-$ was obtained by feeding the components at the molar ratios shown in Table 1 and reacting them by the same procedure as Synthesis Example 1. In (A-2), the content of $R^2$ was 26% by mass, and the content of $R^3$ was 24% by mass.

[Chem. 32]

(A − 2)

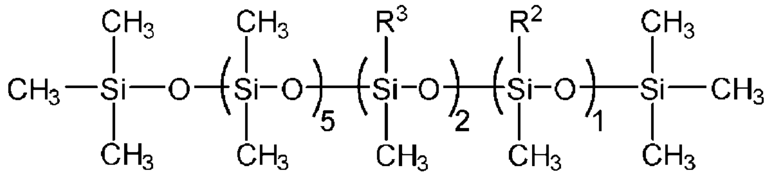

$R^3$: $-C_{10}H_{20}COOH$
$R^2$: $-C_3H_6O(C_2H_4O)_4H$

[Synthesis Example 3]

Organopolysiloxane (A-3) in which in the formula (1), a = 36, b = 2, c = 2, d = 2, e = 0, and f = 0, in the formula (3), i = 3 and j1 = 9, in the formula (4), $L^1$ is $-C_{10}H_{20}-$, and in the formula (5), m = 8 and n = 0 was obtained by feeding the components at the molar ratios shown in Table 1 and reacting them by the same procedure as Synthesis Example 1. In (A-3), the content of $R^1$ having 6 or more carbon atoms was 19% by mass, the content of $R^2$ was 13% by mass, and the content of $R^3$ was 5% by mass.

[Chem. 33]

(A − 3)

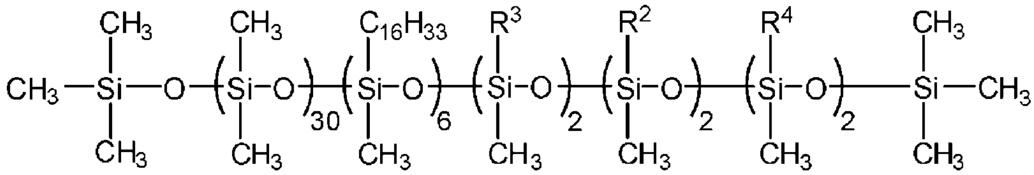

$R^3$: $-C_{10}H_{20}COOH$
$R^2$: $-C_3H_6O(C_2H_4O)_9H$
$R^4$:

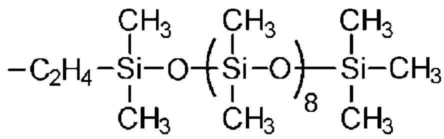

[Synthesis Example 4]

Organopolysiloxane (A-4) in which in the formula (1), a = 15, b = 1, c = 1, d = 0, e = 0, and f = 0, in the formula (2), g = 3 and h = 3, and in the formula (4), $L^1$ is $-C_{10}H_{20}-$ was obtained by feeding the components at the molar ratios shown in Table 1 and reacting them by the same procedure as Synthesis Example 1. In (A-4), the content of $R^2$ was 15% by mass, and the content of $R^3$ was 10% by mass.

[Chem. 34]

(A − 4)

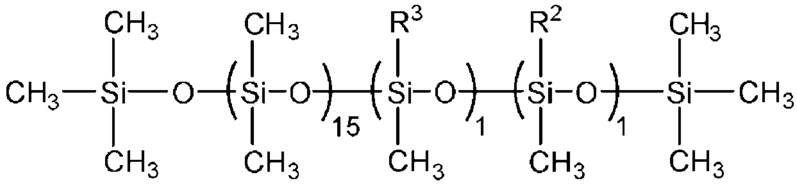

$R^3$ $-C_{10}H_{20}COOH$
$R^2$: $-C_3H_6O(CH_2CH(OH)CH_2O)_3H$

[Synthesis Example 5]

Organopolysiloxane (A-5) in which in the formula (1), a = 60, b = 3, c = 1, d = 0, e = 0, and f = 0, in the formula (3), i = 3 and j1 = 9, and in the formula (4), $L^1$ is $-C_{10}H_{20}-$ was obtained by feeding the components at the molar ratios shown in Table 1 and reacting them by the same procedure as Synthesis Example 1. In (A-5), the content of $R^2$ was 21% by mass, and the content of $R^3$ was 3% by mass.

[Chem. 35]

(A − 5)

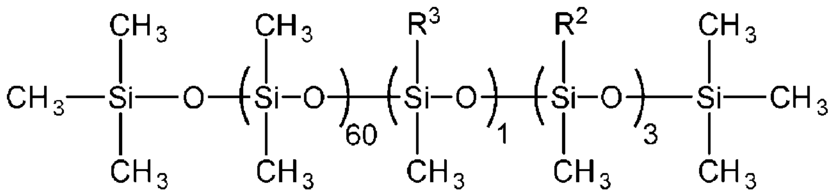

$R^3$: $-C_{10}H_{20}COOH$
$R^2$: $-C_3H_6O(C_2H_4O)_9H$

[Working Examples 1 to 7 and Comparative Examples 1 to 3]

Each oil-in-water type emulsified composition was obtained by mixing the components in the amounts shown in Table 2 by the following manufacturing method.

[Table 2]

| | Component | Working Example 1 | Working Example 2 | Working Example 3 | Working Example 4 | Working Example 5 | Working Example 6 | Working Example 7 | Comparative Example 1 | Comparative Example 2 | Comparative Example 3 |
|---|---|---|---|---|---|---|---|---|---|---|---|
| 1 | Synthesis Example 1 | 2.0 | - | - | - | - | 2.0 | 2.0 | - | - | - |
| | Synthesis Example 2 | - | 2.0 | - | - | - | - | - | - | - | - |
| | Synthesis Example 3 | - | - | 2.0 | - | - | - | - | | - | - |
| | Synthesis Example 4 | - | - | - | 2.0 | - | - | - | - | - | - |
| | Synthesis Example 5 | - | - | - | - | 2.0 | - | - | - | - | - |
| | PEG-11 methyl ether dimethicone | - | - | - | - | - | - | - | 2.0 | - | - |
| | Lauryl polyglyceryl-3 polydimethyl siloxyethyl dimethicone | - | - | - | - | - | - | - | - | 2.0 | - |
| | Carboxydecyl dimethicone | - | - | - | - | 2.0 | - | - | - | - | 2.0 |
| 2 | 1,3-Butylene glycol | 10.0 | 10.0 | 10.0 | 10.0 | 10.0 | 10.0 | 10.0 | 10.0 | 10.0 | 10.0 |
| 3 | Squalane | 20.0 | 20.0 | 20.0 | 20.0 | 20.0 | - | - | 20.0 | 20.0 | 20.0 |
| 4 | Isotridecyl isononanoate | - | - | - | - | - | 20.0 | - | - | - | - |
| 5 | Dimethicone | - | - | - | - | - | - | 20.0 | - | - | - |
| 6 | NaOH (1% aqueous solution) | 4.2 | 4.2 | 4.2 | 4.2 | 4.2 | 4.2 | 4.2 | 4.2 | 4.2 | 4.2 |
| 7 | Ammonium acryloyldimethyltaurate/VP copolymer (5% aqueous solution) (*1) | 20.0 | 20.0 | 20.0 | 20.0 | 20.0 | 20.0 | 20.0 | 20.0 | 20.0 | 20.0 |
| 8 | Purified water | Remainder | Remainder | Remainder | Remainder | Remainder | Remainder | Remainder | Remainder | Remainder | Remainder |
| | Total | 100.0 | 100.0 | 100.0 | 100.0 | 100.0 | 100.0 | 100.0 | 100.0 | 100.0 | 100.0 |
| Evaluation | Emulsification stability | ◎ | ◎ | ◎ | ◎ | ◎ | ◎ | ○ | △ | × | △ |

Components in Table 2:

(*1) Ammonium acryloyldimethyltaurate/VP copolymer; Aristoflex AVC (manufactured by Clariant AG).

(Manufacturing method)

Step 1:

Component (1) and component (2) were mixed, and components (3) to (5) were added thereto and mixed uniformly.

Step 2:

Component (6) and a portion of component (8) were mixed.

Step 3:

The mixture obtained in Step 2 was added to and mixed with the mixture obtained in Step 1 under stirring.

Step 4:

Component (7) and the residue of component (8) were further added to and mixed with the mixture obtained in Step 3 under stirring to obtain an oil-in-water type emulsified composition.

[Working Examples 8 to 10 and Comparative Examples 4 and 5]

The components in the amounts shown in Table 3 were mixed by the manufacturing method below to obtain each oil-in-water type emulsified composition.

[Table 3]

| | Component | Working Example 8 | Working Example 9 | Working Example 10 | Comparative Example 4 | Comparative Example 5 |
|---|---|---|---|---|---|---|
| 1 | Synthesis Example 1 | 2.0 | - | - | - | - |
| | Synthesis Example 2 | - | 2.0 | - | - | - |
| | Synthesis Example 3 | - | - | 2.0 | - | - |
| | PEG-11 methyl ether dimethicone | - | - | - | 2.0 | - |
| | Carboxydecyl dimethicone | - | - | - | - | 2.0 |
| 2 | 1,3-Butylene glycol | 16.0 | 16.0 | 16.0 | 16.0 | 16.0 |
| 3 | Hydrogenated polyisobutene | 10.7 | 10.7 | 10.7 | 10.7 | 10.7 |
| 4 | Isotridecyl isononanoate | 5.0 | 5.0 | 5.0 | 5.0 | 5.0 |
| 5 | Dimethicone | 2.0 | 2.0 | 2.0 | 2.0 | 2.0 |
| 6 | Isostearic acid | 0.3 | 0.3 | 0.3 | 0.3 | 0.3 |
| 7 | Ammonium acryloyldimethyltaurate/VP copolymer (5% aqueous solution) (*1) | 0.6 | 0.6 | 0.6 | 0.6 | 0.6 |
| 8 | NaOH (1% aqueous solution) | 4.2 | 4.2 | 4.2 | 4.2 | 4.2 |
| 9 | PEG-9 dimethicone | 3.4 | 3.4 | 3.4 | 3.4 | 3.4 |
| 10 | Hydrophobized titanium oxide (*2) | 10.0 | 10.0 | 10.0 | 10.0 | 10.0 |
| 11 | Hydrophobized zinc oxide (*3) | 14.0 | 14.0 | 14.0 | 14.0 | 14.0 |
| 12 | Preservative | Moderate amount | Moderate amount | Moderate amount | Moderate amount | Moderate amount |
| 13 | Fragrance | Moderate amount | Moderate amount | Moderate amount | Moderate amount | Moderate amount |
| 14 | Purified water | Remainder | Remainder | Remainder | Remainder | Remainder |
| Total | | 100.0 | 100.0 | 100.0 | 100.0 | 100.0 |
| Evaluation | Emulsification stability | ◎ | ◎ | ◎ | × | × |

Components in Table 3:

(*1) Ammonium acryloyldimethyltaurate/VP copolymer; Aristoflex AVC (manufactured by Clariant AG);
(*2) Hydrophobized titanium oxide; STR-100W-OTS (manufactured by Sakai Chemical Industry Co., Ltd., triethoxycaprylylsilane treatment); and
(*3) Hydrophobized zinc oxide; FINEX-30-OTS (manufactured by Sakai Chemical Industry Co., Ltd., triethoxycaprylylsilane treatment).

(Manufacturing method)

Step 1:

A portion of component (2) and components (9) to (11) were mixed, and the mixture was dispersed in a portion of component (14).

Step 2:

Component (7) was mixed with a portion of component (14) other than the portion used in Step 1.

Step 3:

Component (1) and the residue of component (2) were mixed under stirring, components (3) to (6) were added thereto, and component (8) and the residue of component (14) were further added thereto to obtain a mixture.

Step 4:

The mixture obtained in Step 2 and the dispersion obtained in Step 1 were added to the mixture obtained in Step 3 under stirring, and component (12) and component (13) were then further added thereto and mixed therewith to obtain an oil-in-water type emulsified composition.

[Working Examples 11 and 12]

Each oil-in-water type emulsified composition was obtained by mixing the components in the amounts shown in Table 4 by the following manufacturing method.

[Table 4]

| Component | | Working Example 11 | Working Example 12 |
|---|---|---|---|
| 1 | Synthesis Example 4 | 0.8 | - |
| | Synthesis Example 5 | - | 0.8 |
| 2 | (PEG-15/lauryl dimethicone) crosspolymer (30% mineral oil mixture) | 3.0 | 3.0 |
| 3 | (Vinyl dimethicone/lauryl dimethicone) crosspolymer (30% squalane mixture) | 1.0 | 1.0 |
| 4 | Squalane | 11.0 | 11.0 |
| 5 | 1,3-Butylene glycol | 8.0 | 8.0 |
| 6 | NaOH (1% aqueous solution) | 2.0 | 2.0 |
| 7 | Na citrate | 0.2 | 0.2 |
| 8 | Purified water | Remainder | Remainder |
| Total | | 100.0 | 100.0 |
| Evaluation | Emulsification stability | ◎ | ◎ |

(Manufacturing method)

Step 1:

Components (1) to (4) were mixed uniformly.

Step 2:

Components (5) to (8) were mixed uniformly.

Step 3:

The mixture obtained in Step 2 was added to and emulsified in the mixture obtained in Step 1 under stirring to obtain each water-in-oil type emulsified composition.

[Emulsification stability]

The obtained emulsified compositions were each stored in a 50 mL glass bottle under a condition of 50°C for 3 months. The viscosities of the emulsified composition were measured at 25°C immediately after and one month after the preparation by the method described in JIS K 7117-1: 1999 using a B-type viscometer (TVB-10 type, manufactured by Toki Sangyo Co., Ltd.), and a viscosity change of 0% or more and less than 25% compared to immediately after the preparation was indicated as "◎", a viscosity change of 25% or more and less than 40% was indicated as " ○", a viscosity change of 40% or more and less than 50% was indicated as "△", and a separation between water and oil phases or a viscosity change of 50% or more was indicated as "×". The results are shown in Tables 2 to 4.

It was demonstrated from the results in Tables 2 to 4 that the emulsified compositions of Working Examples 1 to 12 containing a carboxy group-modified organopolysiloxane including a (poly)glycerol group or polyoxyalkylene group have excellent emulsification stability. In contrast, the emulsified compositions obtained by blending polyglycerol-modified or polyether-modified organopolysiloxane and carboxy group-modified silicone of Comparative Examples 1 to 5 lacked storage stability.

[Working Example 13]

Oil-in-water type liquid foundations were obtained by mixing the components in the amounts shown in Table 5 by the following manufacturing method.

[Table 5]

| Component | | Working Example 13 |
|---|---|---|
| 1 | Cetyl PEG/PPG-10/1 dimethicone (HLB 10) | 2.0 |
| 2 | 1,3-Butylene glycol | 10.9 |
| 3 | Squalane | 11.7 |
| 4 | (Vinyl dimethicone/lauryl dimethicone) crosspolymer | 2.0 |
| 5 | Dimethicone (6 cs) | 6.0 |
| 6 | Synthesis Example 1 | 0.3 |
| 7 | NaOH (5% aqueous solution) | 0.4 |
| 8 | Ammonium acryloyldimethyltaurate/VP copolymer (5% aqueous solution) (*1) | 20.0 |
| 9 | Hydrophobized titanium oxide (*2) | 7.0 |
| 10 | Hydrophobized red iron oxide (*3) | 0.3 |
| 11 | Hydrophobized yellow iron oxide (*4) | 1.0 |
| 12 | Hydrophobized black iron oxide (*5) | 0.1 |
| 13 | PEG-9 dimethicone (HLB 10) | 0.3 |
| 14 | Purified water | Remainder |
| Total | | 100.0 |

Components in Table 5:

(*1) Ammonium acryloyldimethyltaurate/VP copolymer; Aristoflex AVC (manufactured by Clariant AG);

(*2) Titanium oxide treated with KF-9901 manufactured by Shin-Etsu Chemical Co., Ltd.;

(*3) Iron oxide (red) treated with KF-9901 manufactured by Shin-Etsu Chemical Co., Ltd.;

(*4) Iron oxide (yellow) treated with KF-9901 manufactured by Shin-Etsu Chemical Co., Ltd.; and

(*5) Iron oxide (black) treated with KF-9901 manufactured by Shin-Etsu Chemical Co., Ltd.

(Manufacturing method)

Step 1:

Component 13 and a portion of component 2 were mixed, and components 9 to 12 were then added thereto and mixed uniformly.

Step 2:

Component 4 and component 5 were mixed uniformly.

Step 3:

Component 1 and the residue of component 2 were mixed uniformly.

Step 4:

Component 3, component 6, and the mixture obtained in Step 2 were mixed uniformly.

Step 5:

Component 7 and a portion of component 14 were mixed uniformly.

Step 6:

The mixture obtained in Step 4 was added to the mixture obtained in Step 3 and emulsified, and the mixture obtained in Step 5 was further added thereto.

Step 7:

Component 8, the residue of component 14, and the mixture obtained in Step 1 were added to the mixture obtained in Step 6 to obtain an oil-in-water (O/W) type liquid foundation,

The oil-in-water type liquid foundation obtained in Example 13 had excellent stability over time, spread easily on the skin, and gave a smooth sense when used. In addition, the resulting makeup film was uniform and had good water resistance and good makeup durability.

## Claims

**1.** A carboxy group-modified organopolysiloxane represented by following formula (1): [Chemical Formula 1]

$$(R_1{}^2SiO_{2/2})_a(R^1R^2SiO_{2/2})_b(R^1R^3SiO_{2/2})_c(R^1R^4SiO_{2/2})_d(R^1SiO_{3/2})_e(SiO_{4/2})_f(R^1{}_3SiO_{1/2})_{2+e+2f} \quad (1)$$

wherein, in the formula, $R^1$s are independently a monovalent hydrocarbon group selected from an alkyl group having 1 to 20 carbon atoms, an aryl group having 6 to 15 carbon atoms, and an aralkyl group having 7 to 15 carbon atoms;

$R^2$s are independently a group represented by following formula (2) or (3):

[Chemical Formula 2]

$$-C_gH_{2g}-O-R^5 \quad (2)$$

wherein, in the formula, $R^5$ is a group in which a hydrogen atom or a monovalent hydrocarbon group having 1 to 30 carbon atoms independently binds to a terminal oxygen atom of the group represented by following formula (2-1):

[Chemical Formula 3]

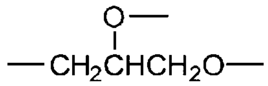 (2-1)

or to a terminal oxygen atom of a plurality of linked groups represented by the formula (2-1), g in the formula (2) is an integer satisfying $0 \leq g \leq 20$, and the number h of the groups represented by the formula (2-1) in $R^5$ is an integer satisfying $1 \leq h \leq 10$;

[Chemical Formula 4]

$$-C_iH_{2i}-O-(C_2H_4O)_{j1}-(C_3H_6O)_{j2}-R^6 \quad (3)$$

wherein, in the formula, $R^6$ is a hydrogen atom or a monovalent hydrocarbon group having 1 to 30 carbon atoms, i is an integer satisfying $0 \leq i \leq 20$, j1 and j2 are integers satisfying $2 \leq j1 \leq 100$ and $0 \leq j2 \leq 100$, respectively, and the order of units grouped by j1 and units grouped by j2 is arbitrary;

$R^3$s are independently a group represented by following formula (4):
[Chemical Formula 5]

$$-L^1-((OR^7)_k-L^2)_z-COOH \quad (4)$$

wherein, in the formula, $L^1$ and $L^2$ are each a divalent linking group having 2 to 20 carbon atoms, $R^7$ is a divalent alkylene group having 2 to 4 carbon atoms, k is an integer satisfying $0 \leq k \leq 100$, and z is 0 or 1;
$R^4$s are independently a group represented by following formula (5), (6), (7), or (8):
[Chemical Formula 6]

$$-(CH_2)_2-C_n-H_{2n}-(SiR^1{}_2O)_m-SiR1_3 \quad (5)$$

$$-(CH_2)_2-C_n-H_{2n}-(SiR^1{}_{m1}-(OSiR^1{}_3)_{3-m1} \quad (6)$$

$$-(CH_2)_2-C_n-H_{2n}-(SiR^1{}_{m1}-(OSiR^1{}_{m2}(OSiR^1{}_3)_{3-m2})_{3m1} \quad (7)$$

$$-(CH_2)_2-C_n-H_{2n}-(SiR^1{}_{m1}-(OSiR^1{}_{m2}(OSiR^1{}_{m3}(OSiR^1{}_3)_{3-m3})_{3-m2})_{3m1} \quad (8)$$

wherein, in the formulae, $R^1$ is as defined above, n and m are integers satisfying $0 \leq n \leq 5$ and $0 \leq m \leq 100$, respectively, and m1, m2, and m3 are integers satisfying $0 \leq m1 \leq 2$, $0 \leq m2 \leq 2$, and $0 \leq m3 \leq 2$; and
a, b, c, d, e, and f are integers satisfying the followings:

$$0 \leq a \leq 100;$$

$$1 \leq b \leq 10;$$

$$1 \leq c \leq 10;$$

$$0 \leq d \leq 10;$$

$$e \geq 0; \text{ and } f \geq 0,$$

and
the order of constituting units grouped by a to f, respectively, is arbitrary.

**2.** The carboxy group-modified organopolysiloxane according to claim 1, wherein the organopolysiloxane is represented by following formula (1-1):

[Chemical Formula 7]

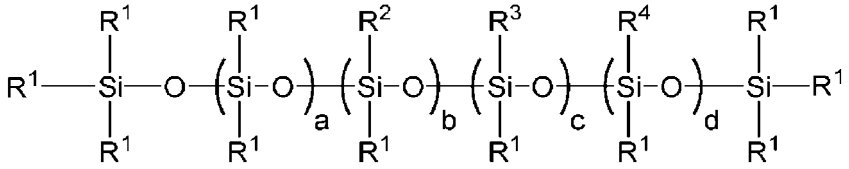

(1-1)

wherein, in the formula, $R^1$, $R^2$, $R^3$, $R^4$, a, b, c, and d are as defined above.

**3.** A cosmetic preparation comprising:

(A) a carboxy group-modified organopolysiloxane represented by the formula (1) according to claim 1;
(B) an oil agent; and
(C) water.

**4.** The cosmetic preparation according to claim 3, wherein the oil agent (B) comprises one or more selected from a silicone oil, a hydrocarbon oil, and a fatty acid ester.

**5.** The cosmetic preparation according to claim 3, wherein the cosmetic preparation is an oil-in-water type emulsified cosmetic preparation.

**INTERNATIONAL SEARCH REPORT**

International application No.
**PCT/JP2024/004637**

**A. CLASSIFICATION OF SUBJECT MATTER**

***C08G 77/38***(2006.01)i; ***A61K 8/06***(2006.01)i; ***A61K 8/31***(2006.01)i; ***A61K 8/37***(2006.01)i; ***A61K 8/81***(2006.01)i; ***A61K 8/89***(2006.01)i; ***A61K 8/92***(2006.01)i; ***A61K 8/891***(2006.01)i; ***A61K 8/894***(2006.01)i; ***A61Q 1/00***(2006.01)i; ***A61Q 1/02***(2006.01)i; ***A61Q 19/00***(2006.01)i; ***C08G 77/46***(2006.01)i; ***C08G 77/48***(2006.01)i; ***C08K 5/5415***(2006.01)i; ***C08L 83/04***(2006.01)i; ***C08L 83/10***(2006.01)i; ***C08L 83/12***(2006.01)i; ***C08L 91/00***(2006.01)i

FI: C08G77/38; A61K8/06; A61K8/31; A61K8/37; A61K8/81; A61K8/89; A61K8/891; A61K8/894; A61K8/92; A61Q1/00; A61Q1/02; A61Q19/00; C08G77/46; C08G77/48; C08K5/5415; C08L83/04; C08L83/10; C08L83/12; C08L91/00

According to International Patent Classification (IPC) or to both national classification and IPC

**B. FIELDS SEARCHED**

Minimum documentation searched (classification system followed by classification symbols)

C08G77/38; A61K8/06; A61K8/31; A61K8/37; A61K8/81; A61K8/89; A61K8/92; A61K8/891; A61K8/894; A61Q1/00; A61Q1/02; A61Q19/00; C08G77/46; C08G77/48; C08K5/5415; C08L83/04; C08L83/10; C08L83/12; C08L91/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| Y | WO 2019/107497 A1 (SHIN-ETSU CHEMICAL CO., LTD.) 06 June 2019 (2019-06-06) claims 1-8, paragraph [0011], examples 1-24 | 1-5 |
| Y | WO 2015/159773 A1 (DOW CORNING TORAY CO., LTD.) 22 October 2015 (2015-10-22) claim 1, paragraphs [0136], [0141], example 1 | 1-5 |
| Y | WO 2020/036065 A1 (DOW TORAY CO., LTD.) 20 February 2020 (2020-02-20) claim 1, paragraphs [0087], [0095], example 1 | 1-5 |
| Y | WO 2022/075372 A1 (DOW TORAY CO., LTD.) 14 April 2022 (2022-04-14) claim 1, examples 1, 5 | 1-5 |
| A | JP 61-223031 A (DOW CORNING CORP.) 03 October 1986 (1986-10-03) entire text | 1-5 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 March 2024** | **02 April 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No.
PCT/JP2024/004637

C. DOCUMENTS CONSIDERED TO BE RELEVANT

| Category* | Citation of document, with indication, where appropriate, of the relevant passages | Relevant to claim No. |
|---|---|---|
| A | JP 05-036441 A (DOW CORNING TORAY SILICONE CO., LTD.) 12 February 1993 (1993-02-12) entire text | 1-5 |
| A | JP 09-111668 A (NIPPON UNICAR CO., LTD.) 28 April 1997 (1997-04-28) entire text | 1-5 |
| A | JP 58-167693 A (TORAY SILICONE CO., LTD.) 03 October 1983 (1983-10-03) entire text | 1-5 |

Form PCT/ISA/210 (second sheet) (July 2022)

**INTERNATIONAL SEARCH REPORT**
**Information on patent family members**

International application No.
**PCT/JP2024/004637**

| Patent document cited in search report | Publication date (day/month/year) | Patent family member(s) | Publication date (day/month/year) |
|---|---|---|---|
| WO 2019/107497 A1 | 06 June 2019 | US 2020/0332065 A1<br>claims 1-8, paragraph [0026], examples 1-24<br>EP 3719056 A1<br>CN 111433256 A<br>KR 10-2020-0093599 A | |
| WO 2015/159773 A1 | 22 October 2015 | US 2017/0035681 A1<br>claim 1, paragraphs [0175], [0188]-[0193], example 1<br>EP 3132789 A1<br>CN 106163495 A<br>KR 10-2016-0136349 A | |
| WO 2020/036065 A1 | 20 February 2020 | US 2021/0322296 A1<br>claim 1, paragraphs [0099], [0109], example 1<br>EP 3838254 A1<br>CN 112739321 A | |
| WO 2022/075372 A1 | 14 April 2022 | US 2023/0372228 A1<br>claim 1, examples 1, 5<br>EP 4227347 A1<br>CN 116096786 A<br>KR 10-2023-0084532 A | |
| JP 61-223031 A | 03 October 1986 | EP 196169 A2<br>entire text | |
| JP 05-036441 A | 12 February 1993 | US 5272021 A<br>entire text<br>EP 525728 A1 | |
| JP 09-111668 A | 28 April 1997 | (Family: none) | |
| JP 58-167693 A | 03 October 1983 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2021075488 A
- WO 2018117172 A
- JP 2020172481 A